# EUROPÄISCHE PATENTSCHRIFT

(11) **EP 1 768 996 B1**
(45) Veröffentlichungstag und Bekanntmachung des Hinweises auf die Patenterteilung: **03.06.2009**
(21) Anmeldenummer: 05761824.1
(22) Anmeldetag: 11.07.2005
(51) Int. Cl.: C07K 14/415

(54) **VARIANTEN DER GRUPPE 1-ALLERGENE AUS POACEAE MIT REDUZIERTER ALLERGENITÄT UND ERHALTENER T-ZELLREAKTIVITÄT**
VARIANTS OF GROUP 1 ALLERGENS OF POACEAE WITH REDUCED ALLERGENICITY AND A MAINTAINED T-CELL REACTIVITY
VARIANTS DES ALLERGENES DU GROUPE 1 ISSUS DE POACEAE PRESENTANT UN EFFET ALLERGENIQUE REDUIT ET UNE REACTIVITE AUX LYMPHOCYTES T CONSERVEE

(30) Priorität: 21.07.2004 DE 102004035337
(43) Veröffentlichungstag der Anmeldung: 04.04.2007
(60) Teilanmeldung: 08014111.2
(73) Patentinhaber: Merck Patent GmbH, 64293 Darmstadt (DE)
(72) Erfinder: FIEBIG, Helmut, 21493 Schwarzenbek (DE); WALD, Martin, 20253 Hamburg (DE); NANDY, Andreas, 22175 Hamburg (DE); KAHLERT, Helga, 22041 Hamburg (DE); WEBER, Bernhard, 21031 Hamburg (DE); CROMWELL, Oliver, 23701 Suesel-Fassendorf (DE)
(86) Internationale Anmeldenummer: PCT/EP2005/007481
(87) Internationale Veröffentlichungsnummer: WO 2006/008018

(56) Entgegenhaltungen:
- WO-A-03/025009
- BHALLA P L: "GENETIC ENGINEERING OF POLLEN ALLERGENS FOR HAYFEVER IMMUNOTHERAPY" EXPERT REVIEW OF VACCINES, FUTURE DRUGS, LONDON, GB, Bd. 2, Nr. 1, Februar 2003 (2003-02), Seiten 75-84, XP009052994 ISSN: 1476-0584
- PETERSEN A ET AL: "Post-translational modifications influence IgE reactivity to the major allergen Phl p 1 of timothy grass pollen." CLINICAL AND EXPERIMENTAL ALLERGY : JOURNAL OF THE BRITISH SOCIETY FOR ALLERGY AND CLINICAL IMMUNOLOGY. MAR 1998, Bd. 28, Nr. 3, März 1998 (1998-03), Seiten 315-321, XP002351773 ISSN: 0954-7894
- SCHRAMM G ET AL: "Allergen engineering: variants of the timothy grass pollen allergen Phl p 5b with reduced IgE-binding capacity but conserved T cell reactivity" JOURNAL OF IMMUNOLOGY, WILLIAMS & WILKINS CO, US, Bd. 162, Nr. 4, 15. Februar 1999 (1999-02-15), Seiten 2406-2414, XP002216586 ISSN: 0022-1767 in der Anmeldung erwähnt
- TAKAI T ET AL: "ENGINEERING OF THE MAJOR HOUSE DUST MITE ALLERGEN DER F2 FOR ALLERGEN-SPECIFIC IMMUNOTHERAPY" NATURE BIOTECHNOLOGY, NATURE PUBLISHING, US, Bd. 15, 15. August 1997 (1997-08-15), Seiten 754-758, XP000783948 ISSN: 1087-0156
- SMITH A M ET AL: "REDUCTION IN IGE BINDING TO ALLERGEN VARIANTS GENERATED BY SITE-DIRECTED MUTAGENESIS: CONTRIBUTION OF DISULFIDE BONDS TO THE ANTIGENIC STRUCTURE OF THE MAJOR HOUSE DUST MITE ALLERGEN DER P 2" MOLECULAR IMMUNOLOGY, ELMSFORD, NY, US, Bd. 33, Nr. 4/5, 1996, Seiten 399-405, XP001010204 ISSN: 0161-5890

### Sequenz-Protokoll

<110> Merck Patent GmbH
<120> Varianten der Gruppe 1-Allergene aus Poaceae mit reduzierter Allergenität und erhaltener T-Zellreaktivität
<130> P 04/107
<140> DE 102004035337.9
   <141> 2004-07-17
<160> 31
<170> PatentIn version 3.1
<210> 1
   <211> 723
   <212> DNA
   <213> Phleum pratense
<220>
   <221> CDS
   <222> (1)..(723)
   <223>
<400> 1
   atc ccc aag gtc ccc ccc ggc ccg aac atc acg gcg acc tac ggc gac       48
   Ile Pro Lys Val Pro Pro Gly Pro Asn Ile Thr Ala Thr Tyr Gly Asp
   1               5                   10                  15
   aag tgg ctg gac gcg aag agc acc tgg tac ggc aag ccg acg gcc gcc       96
   Lys Trp Leu Asp Ala Lys Ser Thr Trp Tyr Gly Lys Pro Thr Ala Ala
               20                  25                  30
   ggt ccc aag gac aac ggc ggc gcg tgc ggg tac aag gac gtg gac aag        144
   Gly Pro Lys Asp Asn Gly Gly Ala Cys Gly Tyr Lys Asp Val Asp Lys
           35                  40                  45
   ccc ccg ttc agc ggc atg acc ggc tgc ggc aac acc ccc atc ttc aag        192
   Pro Pro Phe Ser Gly Met Thr Gly Cys Gly Asn Thr Pro Ile Phe Lys
       50                  55                  60
   tcc ggc cgg ggc tgc ggc tcc tgc ttc gag atc aag tgc acc aag ccc        240
   Ser Gly Arg Gly Cys Gly Ser Cys Phe Glu Ile Lys Cys Thr Lys Pro
   65                  70                  75                  80
   gag gcc tgc tcc ggc gag ccc gtg gtg gtc cac atc acc gac gac aac        288
   Glu Ala Cys Ser Gly Glu Pro Val Val Val His Ile Thr Asp Asp Asn
                   85                  90                  95
   gag gag ccc atc gcc gcg tac cac ttc gac ctc tcc ggc atc gcg ttc        336
   Glu Glu Pro Ile Ala Ala Tyr His Phe Asp Leu Ser Gly Ile Ala Phe
               100                 105                 110
   ggg tcc atg gcc aag aag ggc gac gag cag aag ctg cgc agc gcc ggc        384
   Gly Ser Met Ala Lys Lys Gly Asp Glu Gln Lys Leu Arg Ser Ala Gly
           115                 120                 125
   gag gtg gag atc cag ttc cgc cgc gtc aag tgc aag tac ccg gag ggc        432
   Glu Val Glu Ile Gln Phe Arg Arg Val Lys Cys Lys Tyr Pro Glu Gly
       130                 135                 140
   acc aag gtc acc ttc cac gtg gag aag ggg tcc aac ccc aac tac ctg        480
   Thr Lys Val Thr Phe His Val Glu Lys Gly Ser Asn Pro Asn Tyr Leu
   145                 150                 155                 160
   gcg ctg ctg gtg aag ttt gtc gcc ggc gac ggc gac gtg gtg gcg gtg        528
   Ala Leu Leu Val Lys Phe Val Ala Gly Asp Gly Asp Val Val Ala Val
                   165                 170                 175
   gac atc aag gag aag ggc aag gac aag tgg atc gcg ctc aag gag tcg        576
   Asp Ile Lys Glu Lys Gly Lys Asp Lys Trp Ile Ala Leu Lys Glu Ser
               180                 185                 190
   tgg gga gcc atc tgg agg atc gac acc ccg gag gtg ctc aag ggc ccc        624
   Trp Gly Ala Ile Trp Arg Ile Asp Thr Pro Glu Val Leu Lys Gly Pro
           195                 200                 205
   ttc acc gtc cgc tac acc acc gag ggc ggc acc aag ggc gag gcc aag        672
   Phe Thr Val Arg Tyr Thr Thr Glu Gly Gly Thr Lys Gly Glu Ala Lys
       210                 215                 220
   gac gtc atc ccc gag ggc tgg aag gcc gac acc gcc tac gag tcc aag        720
   Asp Val Ile Pro Glu Gly Trp Lys Ala Asp Thr Ala Tyr Glu Ser Lys
   225                 230                 235                 240
   tga                                                                    723
<210> 2
   <211> 240
   <212> PRT
   <213> Phleum pratense
<400> 2
   Ile Pro Lys Val Pro Pro Gly Pro Asn Ile Thr Ala Thr Tyr Gly Asp
   1               5                   10                  15
   Lys Trp Leu Asp Ala Lys Ser Thr Trp Tyr Gly Lys Pro Thr Ala Ala
               20                  25                  30
   Gly Pro Lys Asp Asn Gly Gly Ala Cys Gly Tyr Lys Asp Val Asp Lys
           35                  40                  45
   Pro Pro Phe Ser Gly Met Thr Gly Cys Gly Asn Thr Pro Ile Phe Lys
       50                  55                  60
   Ser Gly Arg Gly Cys Gly Ser Cys Phe Glu Ile Lys Cys Thr Lys Pro
   65                  70                  75                  80
   Glu Ala Cys Ser Gly Glu Pro Val Val Val His Ile Thr Asp Asp Asn
                   85                  90                  95
   Glu Glu Pro Ile Ala Ala Tyr His Phe Asp Leu Ser Gly Ile Ala Phe
               100                 105                 110
   Gly Ser Met Ala Lys Lys Gly Asp Glu Gln Lys Leu Arg Ser Ala Gly
           115                 120                 125
   Glu Val Glu Ile Gln Phe Arg Arg Val Lys Cys Lys Tyr Pro Glu Gly
       130                 135                 140
   Thr Lys Val Thr Phe His Val Glu Lys Gly Ser Asn Pro Asn Tyr Leu
   145                 150                 155                 160
   Ala Leu Leu Val Lys Phe Val Ala Gly Asp Gly Asp Val Val Ala Val
                   165                 170                 175
   Asp Ile Lys Glu Lys Gly Lys Asp Lys Trp Ile Ala Leu Lys Glu Ser
               180                 185                 190
   Trp Gly Ala Ile Trp Arg Ile Asp Thr Pro Glu Val Leu Lys Gly Pro
           195                 200                 205
   Phe Thr Val Arg Tyr Thr Thr Glu Gly Gly Thr Lys Gly Glu Ala Lys
       210                 215                 220
   Asp Val Ile Pro Glu Gly Trp Lys Ala Asp Thr Ala Tyr Glu Ser Lys
   225                 230                 235                 240
<210> 3
   <211> 723
   <212> DNA
   <213> Phleum pratense
<220>
   <221> CDS
   <222> (1)..(723)
   <223>
<400> 3
   atc ccg aag gtc ccg ccg ggc ccg aac atc acg gcg acc tac ggc gac      48
   Ile Pro Lys Val Pro Pro Gly Pro Asn Ile Thr Ala Thr Tyr Gly Asp
   1               5                   10                  15
   aag tgg ctg gac gcg aag agc acc tgg tac ggc aag ccg acg gcc gcc      96
   Lys Trp Leu Asp Ala Lys Ser Thr Trp Tyr Gly Lys Pro Thr Ala Ala
               20                  25                  30
   ggt ccc aag gac aac ggc ggc gcg agc ggg tac aag gac gtg gac aag     144
   Gly Pro Lys Asp Asn Gly Gly Ala Ser Gly Tyr Lys Asp Val Asp Lys
           35                  40                  45
   ccc ccg ttc agc ggc atg acc ggc tcc ggc aac acc ccc atc ttc aag     192
   Pro Pro Phe Ser Gly Met Thr Gly Ser Gly Asn Thr Pro Ile Phe Lys
       50                  55                  60
   tcc ggc cgg ggc agc ggc tcc tcc ttc gag atc aag agc acc aag ccc     240
   Ser Gly Arg Gly Ser Gly Ser Ser Phe Glu Ile Lys Ser Thr Lys Pro
   65                  70                  75                  80
   gag gcc tcc tcc ggc gag ccc gtg gtg gtc cac atc acc gac gac aac     288
   Glu Ala Ser Ser Gly Glu Pro Val Val Val His Ile Thr Asp Asp Asn
                   85                  90                  95
   gag gag ccc atc gcc gcg tac cac ttc gac ctc tcc ggc atc gcg ttc     336
   Glu Glu Pro Ile Ala Ala Tyr His Phe Asp Leu Ser Gly Ile Ala Phe
               100                 105                 110
   ggg tcc atg gcc aag aag ggc gac gag cag aag ctg cgc agc gcc ggc     384
   Gly Ser Met Ala Lys Lys Gly Asp Glu Gln Lys Leu Arg Ser Ala Gly
           115                 120                 125
   gag gtg gag atc cag ttc cgc cgc gtc aag tcc aag tac ccg gag ggc     432
   Glu Val Glu Ile Gln Phe Arg Arg Val Lys Ser Lys Tyr Pro Glu Gly
       130                 135                 140
   acc aag gtg acc ttc cac gtg gag aag ggg tcc aac ccc aac tac ctg     480
   Thr Lys Val Thr Phe His Val Glu Lys Gly Ser Asn Pro Asn Tyr Leu
   145                 150                 155                 160
   gcg ctg ctg gtg aag ttt gtc gcc ggc gac ggc gac gtg gtg gcg gtg      528
   Ala Leu Leu Val Lys Phe Val Ala Gly Asp Gly Asp Val Val Ala Val
                   165                 170                 175
   gac atc aag gag aag ggc aag gac aag tgg atc gcg ctc aag gag tcg      576
   Asp Ile Lys Glu Lys Gly Lys Asp Lys Trp Ile Ala Leu Lys Glu Ser
               180                 185                 190
   tgg gga gcc atc tgg agg atc gac acc ccg gag gtg ctc aag ggc ccc      624
   Trp Gly Ala Ile Trp Arg Ile Asp Thr Pro Glu Val Leu Lys Gly Pro
           195                 200                 205
   ttc acc gtc cgc tac acc acc gag ggc ggc acc aag ggc gag gcc aag      672
   Phe Thr Val Arg Tyr Thr Thr Glu Gly Gly Thr Lys Gly Glu Ala Lys
       210                 215                 220
   gac gtc atc ccc gag ggc tgg aag gcc gac acc gcc tac gag tcc aag      720
   Asp Val Ile Pro Glu Gly Trp Lys Ala Asp Thr Ala Tyr Glu Ser Lys
   225                 230                 235                 240
   tga                                                                  723
<210> 4
   <211> 240
   <212> PRT
   <213> Phleum pratense
<400> 4
   Ile Pro Lys Val Pro Pro Gly Pro Asn Ile Thr Ala Thr Tyr Gly Asp
   1               5                   10                  15
   Lys Trp Leu Asp Ala Lys Ser Thr Trp Tyr Gly Lys Pro Thr Ala Ala
               20                  25                  30
   Gly Pro Lys Asp Asn Gly Gly Ala Ser Gly Tyr Lys Asp Val Asp Lys
           35                  40                  45
   Pro Pro Phe Ser Gly Met Thr Gly Ser Gly Asn Thr Pro Ile Phe Lys
       50                  55                  60
   Ser Gly Arg Gly Ser Gly Ser Ser Phe Glu Ile Lys Ser Thr Lys Pro
   65                  70                  75                  80
   Glu Ala Ser Ser Gly Glu Pro Val Val Val His Ile Thr Asp Asp Asn
                   85                  90                  95
   Glu Glu Pro Ile Ala Ala Tyr His Phe Asp Leu Ser Gly Ile Ala Phe
               100                 105                 110
   Gly Ser Met Ala Lys Lys Gly Asp Glu Gln Lys Leu Arg Ser Ala Gly
           115                 120                 125
   Glu Val Glu Ile Gln Phe Arg Arg Val Lys Ser Lys Tyr Pro Glu Gly
       130                 135                 140
   Thr Lys Val Thr Phe His Val Glu Lys Gly Ser Asn Pro Asn Tyr Leu
   145                 150                 155                 160
   Ala Leu Leu Val Lys Phe Val Ala Gly Asp Gly Asp Val Val Ala Val
                   165                 170                 175
   Asp Ile Lys Glu Lys Gly Lys Asp Lys Trp Ile Ala Leu Lys Glu Ser
               180                 185                 190
   Trp Gly Ala Ile Trp Arg Ile Asp Thr Pro Glu Val Leu Lys Gly Pro
           195                 200                 205
   Phe Thr Val Arg Tyr Thr Thr Glu Gly Gly Thr Lys Gly Glu Ala Lys
       210                 215                 220
   Asp Val Ile Pro Glu Gly Trp Lys Ala Asp Thr Ala Tyr Glu Ser Lys
   225                 230                 235                 240
<210> 5
   <211> 705
   <212> DNA
   <213> Phleum pratense
<220>
   <221> CDS
   <222> (1)..(705)
   <223>
<400> 5
   ggc ccg aac atc acg gcg acc tac ggc gac aag tgg ctg gac gcg aag      48
   Gly Pro Asn Ile Thr Ala Thr Tyr Gly Asp Lys Trp Leu Asp Ala Lys
   1               5                   10                  15
   agc acc tgg tac ggc aag ccg acg gcc gcc ggt ccc aag gac aac ggc      96
   Ser Thr Trp Tyr Gly Lys Pro Thr Ala Ala Gly Pro Lys Asp Asn Gly
               20                  25                  30
   ggc gcg agc ggg tac aag gac gtg gac aag ccc ccg ttc agc ggc atg      144
   Gly Ala Ser Gly Tyr Lys Asp Val Asp Lys Pro Pro Phe Ser Gly Met
           35                  40                  45
   acc ggc tcc ggc aac acc ccc atc ttc aag tcc ggc cgg ggc agc ggc      192
   Thr Gly Ser Gly Asn Thr Pro Ile Phe Lys Ser Gly Arg Gly Ser Gly
       50                  55                  60
   tcc tcc ttc gag atc aag agc acc aag ccc gag gcc tcc tcc ggc gag      240
   Ser Ser Phe Glu Ile Lys Ser Thr Lys Pro Glu Ala Ser Ser Gly Glu
   65                  70                  75                  80
   ccc gtg gtg gtc cac atc acc gac gac aac gag gag ccc atc gcc gcg      288
   Pro Val Val Val His Ile Thr Asp Asp Asn Glu Glu Pro Ile Ala Ala
                   85                  90                  95
   tac cac ttc gac ctc tcc ggc atc gcg ttc ggg tcc atg gcc aag aag      336
   Tyr His Phe Asp Leu Ser Gly Ile Ala Phe Gly Ser Met Ala Lys Lys
               100                 105                 110
   ggc gac gag cag aag ctg cgc agc gcc ggc gag gtg gag atc cag ttc      384
   Gly Asp Glu Gln Lys Leu Arg Ser Ala Gly Glu Val Glu Ile Gln Phe
           115                 120                 125
   cgc cgc gtc aag tcc aag tac ccg gag ggc acc aag gtg acc ttc cac      432
   Arg Arg Val Lys Ser Lys Tyr Pro Glu Gly Thr Lys Val Thr Phe His
       130                 135                 140
   gtg gag aag ggg tcc aac ccc aac tac ctg gcg ctg ctg gtg aag ttt      480
   Val Glu Lys Gly Ser Asn Pro Asn Tyr Leu Ala Leu Leu Val Lys Phe
   145                 150                 155                 160
   gtc gcc ggc gac ggc gac gtg gtg gcg gtg gac atc aag gag aag ggc      528
   Val Ala Gly Asp Gly Asp Val Val Ala Val Asp Ile Lys Glu Lys Gly
                   165                 170                 175
   aag gac aag tgg atc gcg ctc aag gag tcg tgg gga gcc atc tgg agg      576
   Lys Asp Lys Trp Ile Ala Leu Lys Glu Ser Trp Gly Ala Ile Trp Arg
               180                 185                 190
   atc gac acc ccg gag gtg ctc aag ggc ccc ttc acc gtc cgc tac acc      624
   Ile Asp Thr Pro Glu Val Leu Lys Gly Pro Phe Thr Val Arg Tyr Thr
           195                 200                 205
   acc gag ggc ggc acc aag ggc gag gcc aag gac gtc atc ccc gag ggc      672
   Thr Glu Gly Gly Thr Lys Gly Glu Ala Lys Asp Val Ile Pro Glu Gly
       210                 215                 220
   tgg aag gcc gac acc gcc tac gag tcc aag tga                          705
   Trp Lys Ala Asp Thr Ala Tyr Glu Ser Lys
   225                 230
<210> 6
   <211> 234
   <212> PRT
   <213> Phleum pratense
<400> 6
   Gly Pro Asn Ile Thr Ala Thr Tyr Gly Asp Lys Trp Leu Asp Ala Lys
   1               5                   10                  15
   Ser Thr Trp Tyr Gly Lys Pro Thr Ala Ala Gly Pro Lys Asp Asn Gly
               20                  25                  30
   Gly Ala Ser Gly Tyr Lys Asp Val Asp Lys Pro Pro Phe Ser Gly Met
           35                  40                  45
   Thr Gly Ser Gly Asn Thr Pro Ile Phe Lys Ser Gly Arg Gly Ser Gly
       50                  55                  60
   Ser Ser Phe Glu Ile Lys Ser Thr Lys Pro Glu Ala Ser Ser Gly Glu
   65                  70                  75                  80
   Pro Val Val Val His Ile Thr Asp Asp Asn Glu Glu Pro Ile Ala Ala
                   85                  90                  95
   Tyr His Phe Asp Leu Ser Gly Ile Ala Phe Gly Ser Met Ala Lys Lys
               100                 105                 110
   Gly Asp Glu Gln Lys Leu Arg Ser Ala Gly Glu Val Glu Ile Gln Phe
           115                 120                 125
   Arg Arg Val Lys Ser Lys Tyr Pro Glu Gly Thr Lys Val Thr Phe His
       130                 135                 140
   Val Glu Lys Gly Ser Asn Pro Asn Tyr Leu Ala Leu Leu Val Lys Phe
   145                 150                 155                 160
   Val Ala Gly Asp Gly Asp Val Val Ala Val Asp Ile Lys Glu Lys Gly
                   165                 170                 175
   Lys Asp Lys Trp Ile Ala Leu Lys Glu Ser Trp Gly Ala Ile Trp Arg
               180                 185                 190
   Ile Asp Thr Pro Glu Val Leu Lys Gly Pro Phe Thr Val Arg Tyr Thr
           195                 200                 205
   Thr Glu Gly Gly Thr Lys Gly Glu Ala Lys Asp Val Ile Pro Glu Gly
       210                 215                 220
   Trp Lys Ala Asp Thr Ala Tyr Glu Ser Lys
   225                 230
<210> 7
   <211> 633
   <212> DNA
   <213> Phleum pratense
<220>
   <221> CDS
   <222> (1)..(633)
   <223>
<400> 7
   gcc gcc ggt ccc aag gac aac ggc ggc gcg agc ggg tac aag gac gtg        48
   Ala Ala Gly Pro Lys Asp Asn Gly Gly Ala Ser Gly Tyr Lys Asp Val
   1               5                   10                  15
   gac aag ccc ccg ttc agc ggc atg acc ggc tcc ggc aac acc ccc atc        96
   Asp Lys Pro Pro Phe Ser Gly Met Thr Gly Ser Gly Asn Thr Pro Ile
               20                  25                  30
   ttc aag tcc ggc cgg ggc agc ggc tcc tcc ttc gag atc aag agc acc       144
   Phe Lys Ser Gly Arg Gly Ser Gly Ser Ser Phe Glu Ile Lys Ser Thr
           35                  40                  45
   aag ccc gag gcc tcc tcc ggc gag ccc gtg gtg gtc cac atc acc gac       192
   Lys Pro Glu Ala Ser Ser Gly Glu Pro Val Val Val His Ile Thr Asp
       50                  55                  60
   gac aac gag gag ccc atc gcc gcg tac cac ttc gac ctc tcc ggc atc       240
   Asp Asn Glu Glu Pro Ile Ala Ala Tyr His Phe Asp Leu Ser Gly Ile
   65                  70                  75                  80
   gcg ttc ggg tcc atg gcc aag aag ggc gac gag cag aag ctg cgc agc       288
   Ala Phe Gly Ser Met Ala Lys Lys Gly Asp Glu Gln Lys Leu Arg Ser
                   85                  90                  95
   gcc ggc gag gtg gag atc cag ttc cgc cgc gtc aag tcc aag tac ccg       336
   Ala Gly Glu Val Glu Ile Gln Phe Arg Arg Val Lys Ser Lys Tyr Pro
               100                 105                 110
   gag ggc acc aag gtg acc ttc cac gtg gag aag ggg tcc aac ccc aac       384
   Glu Gly Thr Lys Val Thr Phe His Val Glu Lys Gly Ser Asn Pro Asn
           115                 120                 125
   tac ctg gcg ctg ctg gtg aag ttt gtc gcc ggc gac ggc gac gtg gtg       432
   Tyr Leu Ala Leu Leu Val Lys Phe Val Ala Gly Asp Gly Asp Val Val
       130                 135                 140
   gcg gtg gac atc aag gag aag ggc aag gac aag tgg atc gcg ctc aag       480
   Ala Val Asp Ile Lys Glu Lys Gly Lys Asp Lys Trp Ile Ala Leu Lys
   145                 150                 155                 160
   gag tcg tgg gga gcc atc tgg agg atc gac acc ccg gag gtg ctc aag       528
   Glu Ser Trp Gly Ala Ile Trp Arg Ile Asp Thr Pro Glu Val Leu Lys
                   165                 170                 175
   ggc ccc ttc acc gtc cgc tac acc acc gag ggc ggc acc aag ggc gag       576
   Gly Pro Phe Thr Val Arg Tyr Thr Thr Glu Gly Gly Thr Lys Gly Glu
               180                 185                 190
   gcc aag gac gtc atc ccc gag ggc tgg aag gcc gac acc gcc tac gag       624
   Ala Lys Asp Val Ile Pro Glu Gly Trp Lys Ala Asp Thr Ala Tyr Glu
           195                 200                 205
   tcc aag tga                                                           633
   Ser Lys
       210
<210> 8
   <211> 210
   <212> PRT
   <213> Phleum pratense
<400> 8
   Ala Ala Gly Pro Lys Asp Asn Gly Gly Ala Ser Gly Tyr Lys Asp Val
   1               5                   10                  15
   Asp Lys Pro Pro Phe Ser Gly Met Thr Gly Ser Gly Asn Thr Pro Ile
               20                  25                  30
   Phe Lys Ser Gly Arg Gly Ser Gly Ser Ser Phe Glu Ile Lys Ser Thr
           35                  40                  45
   Lys Pro Glu Ala Ser Ser Gly Glu Pro Val Val Val His Ile Thr Asp
       50                  55                  60
   Asp Asn Glu Glu Pro Ile Ala Ala Tyr His Phe Asp Leu Ser Gly Ile
   65                  70                  75                  80
   Ala Phe Gly Ser Met Ala Lys Lys Gly Asp Glu Gln Lys Leu Arg Ser
                   85                  90                  95
   Ala Gly Glu Val Glu Ile Gln Phe Arg Arg Val Lys Ser Lys Tyr Pro
               100                 105                 110
   Glu Gly Thr Lys Val Thr Phe His Val Glu Lys Gly Ser Asn Pro Asn
           115                 120                 125
   Tyr Leu Ala Leu Leu Val Lys Phe Val Ala Gly Asp Gly Asp Val Val
   130 135 140
   Ala Val Asp Ile Lys Glu Lys Gly Lys Asp Lys Trp Ile Ala Leu Lys
   145 150 155 160
   Glu Ser Trp Gly Ala Ile Trp Arg Ile Asp Thr Pro Glu Val Leu Lys
   165 170 175
   Gly Pro Phe Thr Val Arg Tyr Thr Thr Glu Gly Gly Thr Lys Gly Glu
   180 185 190
   Ala Lys Asp Val Ile Pro Glu Gly Trp Lys Ala Asp Thr Ala Tyr Glu
   195 200 205
   Ser Lys
   210
<210> 9
   <211> 684
   <212> DNA
   <213> Phleum pratense
<220>
   <221> CDS
   <222> (1)..(684)
   <223>
<400> 9
   atc ccg aag gtc ccg ccg ggc ccg aac atc acg gcg acc tac ggc gac 48
   Ile Pro Lys Val Pro Pro Gly Pro Asn Ile Thr Ala Thr Tyr Gly Asp
   1 5 10 15
   aag tgg ctg gac gcg aag agc acc tgg tac ggc aag ccg acg gcc gcc 96
   Lys Trp Leu Asp Ala Lys Ser Thr Trp Tyr Gly Lys Pro Thr Ala Ala
   20 25 30
   ggt ccc aag gac aac ggc ggc gcg agc ggg tac aag gac gtg gac aag 144
   Gly Pro Lys Asp Asn Gly Gly Ala Ser Gly Tyr Lys Asp Val Asp Lys
   35 40 45
   ccc ccg ttc agc ggc atg acc ggc tcc ggc aac acc ccc atc ttc aag 192
   Pro Pro Phe Ser Gly Met Thr Gly Ser Gly Asn Thr Pro Ile Phe Lys
   50 55 60
   tcc ggc cgg ggc agc ggc tcc tcc ttc gag atc aag agc acc aag ccc       240
   Ser Gly Arg Gly Ser Gly Ser Ser Phe Glu Ile Lys Ser Thr Lys Pro
   65                  70                  75                  80
   gag gcc tcc tcc ggc gag ccc gtg gtg gtc cac ttc gac ctc tcc ggc       288
   Glu Ala Ser Ser Gly Glu Pro Val Val Val His Phe Asp Leu Ser Gly
                   85                  90                  95
   atc gcg ttc ggg tcc atg gcc aag aag ggc gac gag cag aag ctg cgc       336
   Ile Ala Phe Gly Ser Met Ala Lys Lys Gly Asp Glu Gln Lys Leu Arg
               100                 105                 110
   agc gcc ggc gag gtg gag atc cag ttc cgc cgc gtc aag tcc aag tac       384
   Ser Ala Gly Glu Val Glu Ile Gln Phe Arg Arg Val Lys Ser Lys Tyr
           115                 120                 125
   ccg gag ggc acc aag gtg acc ttc cac gtg gag aag ggg tcc aac ccc       432
   Pro Glu Gly Thr Lys Val Thr Phe His Val Glu Lys Gly Ser Asn Pro
       130                 135                 140
   aac tac ctg gcg ctg ctg gtg aag ttt gtc gcc ggc gac ggc gac gtg       480
   Asn Tyr Leu Ala Leu Leu Val Lys Phe Val Ala Gly Asp Gly Asp Val
   145                 150                 155                 160
   gtg gcg gtg gac atc aag gag aag ggc aag gac aag tgg atc gcg ctc       528
   Val Ala Val Asp Ile Lys Glu Lys Gly Lys Asp Lys Trp Ile Ala Leu
                   165                 170                 175
   aag gag tcg tgg gga gcc atc tgg agg atc gac acc ccg gag gtg ctc       576
   Lys Glu Ser Trp Gly Ala Ile Trp Arg Ile Asp Thr Pro Glu Val Leu
               180                 185                 190
   aag ggc ccc ttc acc gtc cgc tac acc acc gag ggc ggc acc aag ggc       624
   Lys Gly Pro Phe Thr Val Arg Tyr Thr Thr Glu Gly Gly Thr Lys Gly
           195                 200                 205
   gag gcc aag gac gtc atc ccc gag ggc tgg aag gcc gac acc gcc tac       672
   Glu Ala Lys Asp Val Ile Pro Glu Gly Trp Lys Ala Asp Thr Ala Tyr
       210                 215                 220
   gag tcc aag tga                                                       684
   Glu Ser Lys
   225
<210> 10
   <211> 227
   <212> PRT
   <213> Phleum pratense
<400> 10
   Ile Pro Lys Val Pro Pro Gly Pro Asn Ile Thr Ala Thr Tyr Gly Asp
   1               5                   10                  15
   Lys Trp Leu Asp Ala Lys Ser Thr Trp Tyr Gly Lys Pro Thr Ala Ala
   20 25 30
   Gly Pro Lys Asp Asn Gly Gly Ala Ser Gly Tyr Lys Asp Val Asp Lys
   35 40 45
   Pro Pro Phe Ser Gly Met Thr Gly Ser Gly Asn Thr Pro Ile Phe Lys
   50 55 60
   Ser Gly Arg Gly Ser Gly Ser Ser Phe Glu Ile Lys Ser Thr Lys Pro
   65 70 75 80
   Glu Ala Ser Ser Gly Glu Pro Val Val Val His Phe Asp Leu Ser Gly
   85 90 95
   Ile Ala Phe Gly Ser Met Ala Lys Lys Gly Asp Glu Gln Lys Leu Arg
   100 105 110
   Ser Ala Gly Glu Val Glu Ile Gln Phe Arg Arg Val Lys Ser Lys Tyr
   115 120 125
   Pro Glu Gly Thr Lys Val Thr Phe His Val Glu Lys Gly Ser Asn Pro
   130 135 140
   Asn Tyr Leu Ala Leu Leu Val Lys Phe Val Ala Gly Asp Gly Asp Val
   145 150 155 160
   Val Ala Val Asp Ile Lys Glu Lys Gly Lys Asp Lys Trp Ile Ala Leu
   165 170 175
   Lys Glu Ser Trp Gly Ala Ile Trp Arg Ile Asp Thr Pro Glu Val Leu
   180 185 190
   Lys Gly Pro Phe Thr Val Arg Tyr Thr Thr Glu Gly Gly Thr Lys Gly
   195 200 205
   Glu Ala Lys Asp Val Ile Pro Glu Gly Trp Lys Ala Asp Thr Ala Tyr
   210 215 220
   Glu Ser Lys
   225
<210> 11
   <211> 708
   <212> DNA
   <213> Phleum pratense
<220>
   <221> CDS
   <222> (1)..(708)
   <223>
<400> 11
   atc ccg aag gtc ccg ccg ggc ccg aac atc acg gcg acc tac ggc gac        48
   Ile Pro Lys Val Pro Pro Gly Pro Asn Ile Thr Ala Thr Tyr Gly Asp
   1               5                   10                  15
   aag tgg ctg gac gcg aag agc acc tgg tac ggc aag ccg acg gcc gcc        96
   Lys Trp Leu Asp Ala Lys Ser Thr Trp Tyr Gly Lys Pro Thr Ala Ala
               20                  25                  30
   ggt ccc aag gac aac ggc ggc gcg agc ggg tac aag gac gtg gac aag       144
   Gly Pro Lys Asp Asn Gly Gly Ala Ser Gly Tyr Lys Asp Val Asp Lys
           35                  40                  45
   ccc ccg ttc agc ggc atg acc ggc tcc ggc aac acc ccc atc ttc aag       192
   Pro Pro Phe Ser Gly Met Thr Gly Ser Gly Asn Thr Pro Ile Phe Lys
       50                  55                  60
   tcc ggc cgg ggc agc ggc tcc tcc ttc gag atc aag agc acc aag ccc       240
   Ser Gly Arg Gly Ser Gly Ser Ser Phe Glu Ile Lys Ser Thr Lys Pro
   65                  70                  75                  80
   gag gcc tcc tcc ggc gag ccc gtg gtg gtc cac atc acc gac gac aac       288
   Glu Ala Ser Ser Gly Glu Pro Val Val Val His Ile Thr Asp Asp Asn
                   85                  90                  95
   gag gag ccc atc gcc gcg tac cac ttc gac ctc tcc ggc atc gcg ttc       336
   Glu Glu Pro Ile Ala Ala Tyr His Phe Asp Leu Ser Gly Ile Ala Phe
               100                 105                 110
   ggg tcc gac gag cag aag ctg cgc agc gcc ggc gag gtg gag atc cag       384
   Gly Ser Asp Glu Gln Lys Leu Arg Ser Ala Gly Glu Val Glu Ile Gln
           115                 120                 125
   ttc cgc cgc gtc aag tcc aag tac ccg gag ggc acc aag gtg acc ttc       432
   Phe Arg Arg Val Lys Ser Lys Tyr Pro Glu Gly Thr Lys Val Thr Phe
       130                 135                 140
   cac gtg gag aag ggg tcc aac ccc aac tac ctg gcg ctg ctg gtg aag       480
   His Val Glu Lys Gly Ser Asn Pro Asn Tyr Leu Ala Leu Leu Val Lys
   145                 150                 155                 160
   ttt gtc gcc ggc gac ggc gac gtg gtg gcg gtg gac atc aag gag aag       528
   Phe Val Ala Gly Asp Gly Asp Val Val Ala Val Asp Ile Lys Glu Lys
                   165                 170                 175
   ggc aag gac aag tgg atc gcg ctc aag gag tcg tgg gga gcc atc tgg       576
   Gly Lys Asp Lys Trp Ile Ala Leu Lys Glu Ser Trp Gly Ala Ile Trp
               180                 185                 190
   agg atc gac acc ccg gag gtg ctc aag ggc ccc ttc acc gtc cgc tac        624
   Arg Ile Asp Thr Pro Glu Val Leu Lys Gly Pro Phe Thr Val Arg Tyr
       195                 200                     205
   acc acc gag ggc ggc acc aag ggc gag gcc aag gac gtc atc ccc gag        672
   Thr Thr Glu Gly Gly Thr Lys Gly Glu Ala Lys Asp Val Ile Pro Glu
       210                 215                 220
   ggc tgg aag gcc gac acc gcc tac gag tcc aag tga                        708
   Gly Trp Lys Ala Asp Thr Ala Tyr Glu Ser Lys
   225                 230                 235
<210> 12
   <211> 235
   <212> PRT
   <213> Phleum pratense
<400> 12
   Ile Pro Lys Val Pro Pro Gly Pro Asn Ile Thr Ala Thr Tyr Gly Asp
   1               5                   10                  15
   Lys Trp Leu Asp Ala Lys Ser Thr Trp Tyr Gly Lys Pro Thr Ala Ala
               20                  25                  30
   Gly Pro Lys Asp Asn Gly Gly Ala Ser Gly Tyr Lys Asp Val Asp Lys
           35                  40                  45
   Pro Pro Phe Ser Gly Met Thr Gly Ser Gly Asn Thr Pro Ile Phe Lys
       50                  55                  60
   Ser Gly Arg Gly Ser Gly Ser Ser Phe Glu Ile Lys Ser Thr Lys Pro
   65                  70                  75                  80
   Glu Ala Ser Ser Gly Glu Pro Val Val Val His Ile Thr Asp Asp Asn
                   85                  90                  95
   Glu Glu Pro Ile Ala Ala Tyr His Phe Asp Leu Ser Gly Ile Ala Phe
               100                 105                 110
   Gly Ser Asp Glu Gln Lys Leu Arg Ser Ala Gly Glu Val Glu Ile Gln
           115                 120                 125
   Phe Arg Arg Val Lys Ser Lys Tyr Pro Glu Gly Thr Lys Val Thr Phe
       130                 135                 140
   His Val Glu Lys Gly Ser Asn Pro Asn Tyr Leu Ala Leu Leu Val Lys
   145 150 155 160
   Phe Val Ala Gly Asp Gly Asp Val Val Ala Val Asp Ile Lys Glu Lys
   165 170 175
   Gly Lys Asp Lys Trp Ile Ala Leu Lys Glu Ser Trp Gly Ala Ile Trp
   180 185 190
   Arg Ile Asp Thr Pro Glu Val Leu Lys Gly Pro Phe Thr Val Arg Tyr
   195 200 205
   Thr Thr Glu Gly Gly Thr Lys Gly Glu Ala Lys Asp Val Ile Pro Glu
   210 215 220
   Gly Trp Lys Ala Asp Thr Ala Tyr Glu Ser Lys
   225 230 235
<210> 13
   <211> 690
   <212> DNA
   <213> Phleum pratense
<220>
   <221> CDS
   <222> (1)..(690)
   <223>
<400> 13
   atc ccg aag gtc ccg ccg ggc ccg aac atc acg gcg acc tac ggc gac 48
   Ile Pro Lys Val Pro Pro Gly Pro Asn Ile Thr Ala Thr Tyr Gly Asp
   1 5 10 15
   aag tgg ctg gac gcg aag agc acc tgg tac ggc aag ccg acg gcc gcc 96
   Lys Trp Leu Asp Ala Lys Ser Thr Trp Tyr Gly Lys Pro Thr Ala Ala
   20 25 30
   ggt ccc aag gac aac ggc ggc gcg agc ggg tac aag gac gtg gac aag 144
   Gly Pro Lys Asp Asn Gly Gly Ala Ser Gly Tyr Lys Asp Val Asp Lys
   35 40 45
   ccc ccg ttc agc ggc atg acc ggc tcc ggc aac acc ccc atc ttc aag 192
   Pro Pro Phe Ser Gly Met Thr Gly Ser Gly Asn Thr Pro Ile Phe Lys
   50 55 60
   tcc ggc cgg ggc agc ggc tcc tcc ttc gag atc aag agc acc aag ccc 240
   Ser Gly Arg Gly Ser Gly Ser Ser Phe Glu Ile Lys Ser Thr Lys Pro
   65                  70                  75                  80
   gag gcc tcc tcc ggc gag ccc gtg gtg gtc cac atc acc gac gac aac       288
   Glu Ala Ser Ser Gly Glu Pro Val Val Val His Ile Thr Asp Asp Asn
                   85                  90                  95
   gag gag ccc atc gcc gcg tac cac ttc gac ctc tcc ggc atc gcg ttc       336
   Glu Glu Pro Ile Ala Ala Tyr His Phe Asp Leu Ser Gly Ile Ala Phe
               100                 105                 110
   ggg tcc atg gcc aag aag ggc gac gag cag aag ctg cgc agc gcc ggc       384
   Gly Ser Met Ala Lys Lys Gly Asp Glu Gln Lys Leu Arg Ser Ala Gly
           115                 120                 125
   gag gtg gag atc cag ttc cgc cgc gtc aag tcc aag tac ccg gag ggc       432
   Glu Val Glu Ile Gln Phe Arg Arg Val Lys Ser Lys Tyr Pro Glu Gly
       130                 135                 140
   acc aag gtg acc ttc cac gtg gag aag ggg tcc aac ccc aac tac ctg       480
   Thr Lys Val Thr Phe His Val Glu Lys Gly Ser Asn Pro Asn Tyr Leu
   145                 150                 155                 160
   gcg ctg ctg gtg aag ttt gtc gcc ggc gac ggc gac gtg gtg tgg atc       528
   Ala Leu Leu Val Lys Phe Val Ala Gly Asp Gly Asp Val Val Trp Ile
                   165                 170                 175
   gcg ctc aag gag tcg tgg gga gcc atc tgg agg atc gac acc ccg gag       576
   Ala Leu Lys Glu Ser Trp Gly Ala Ile Trp Arg Ile Asp Thr Pro Glu
               180                 185                 190
   gtg ctc aag ggc ccc ttc acc gtc cgc tac acc acc gag ggc ggc acc       624
   Val Leu Lys Gly Pro Phe Thr Val Arg Tyr Thr Thr Glu Gly Gly Thr
           195                 200                 205
   aag ggc gag gcc aag gac gtc atc ccc gag ggc tgg aag gcc gac acc       672
   Lys Gly Glu Ala Lys Asp Val Ile Pro Glu Gly Trp Lys Ala Asp Thr
       210                 215                 220
   gcc tac gag tcc aag tga                                               690
   Ala Tyr Glu Ser Lys
   225
<210> 14
   <211> 229
   <212> PRT
   <213> Phleum pratense
<400> 14
   Ile Pro Lys Val Pro Pro Gly Pro Asn Ile Thr Ala Thr Tyr Gly Asp
   1               5                   10                  15
   Lys Trp Leu Asp Ala Lys Ser Thr Trp Tyr Gly Lys Pro Thr Ala Ala
               20                  25                  30
   Gly Pro Lys Asp Asn Gly Gly Ala Ser Gly Tyr Lys Asp Val Asp Lys
   35 40 45
   Pro Pro Phe Ser Gly Met Thr Gly Ser Gly Asn Thr Pro Ile Phe Lys
   50 55 60
   Ser Gly Arg Gly Ser Gly Ser Ser Phe Glu Ile Lys Ser Thr Lys Pro
   65 70 75 80
   Glu Ala Ser Ser Gly Glu Pro Val Val Val His Ile Thr Asp Asp Asn
   85 90 95
   Glu Glu Pro Ile Ala Ala Tyr His Phe Asp Leu Ser Gly Ile Ala Phe
   100 105 110
   Gly Ser Met Ala Lys Lys Gly Asp Glu Gln Lys Leu Arg Ser Ala Gly
   115 120 125
   Glu Val Glu Ile Gln Phe Arg Arg Val Lys Ser Lys Tyr Pro Glu Gly
   130 135 140
   Thr Lys Val Thr Phe His Val Glu Lys Gly Ser Asn Pro Asn Tyr Leu
   145 150 155 160
   Ala Leu Leu Val Lys Phe Val Ala Gly Asp Gly Asp Val Val Trp Ile
   165 170 175
   Ala Leu Lys Glu Ser Trp Gly Ala Ile Trp Arg Ile Asp Thr Pro Glu
   180 185 190
   Val Leu Lys Gly Pro Phe Thr Val Arg Tyr Thr Thr Glu Gly Gly Thr
   195 200 205
   Lys Gly Glu Ala Lys Asp Val Ile Pro Glu Gly Trp Lys Ala Asp Thr
   210 215 220
   Ala Tyr Glu Ser Lys
   225
<210> 15
   <211> 699
   <212> DNA
   <213> Phleum pratense
<220>
   <221> CDS
   <222> (1)..(699)
   <223>
<400> 15
   atc ccg aag gtc ccg ccg ggc ccg aac atc acg gcg acc tac ggc gac      48
   Ile Pro Lys Val Pro Pro Gly Pro Asn Ile Thr Ala Thr Tyr Gly Asp
   1               5                   10                  15
   aag tgg ctg gac gcg aag agc acc tgg tac ggc aag ccg acg gcc gcc      96
   Lys Trp Leu Asp Ala Lys Ser Thr Trp Tyr Gly Lys Pro Thr Ala Ala
               20                  25                  30
   ggt ccc aag gac aac ggc ggc gcg agc ggg tac aag gac gtg gac aag     144
   Gly Pro Lys Asp Asn Gly Gly Ala Ser Gly Tyr Lys Asp Val Asp Lys
           35                  40                  45
   ccc ccg ttc agc ggc atg acc ggc tcc ggc aac acc ccc atc ttc aag     192
   Pro Pro Phe Ser Gly Met Thr Gly Ser Gly Asn Thr Pro Ile Phe Lys
       50                  55                  60
   tcc ggc cgg ggc agc ggc tcc tcc ttc gag atc aag agc acc aag ccc     240
   Ser Gly Arg Gly Ser Gly Ser Ser Phe Glu Ile Lys Ser Thr Lys Pro
   65                  70                  75                  80
   gag gcc tcc tcc ggc gag ccc gtg gtg gtc cac atc acc gac gac aac     288
   Glu Ala Ser Ser Gly Glu Pro Val Val Val His Ile Thr Asp Asp Asn
                   85                  90                  95
   gag gag ccc atc gcc gcg tac cac ttc gac ctc tcc ggc atc gcg ttc     336
   Glu Glu Pro Ile Ala Ala Tyr His Phe Asp Leu Ser Gly Ile Ala Phe
               100                 105                 110
   ggg tcc atg gcc aag aag ggc gac gag cag aag ctg cgc agc gcc ggc     384
   Gly Ser Met Ala Lys Lys Gly Asp Glu Gln Lys Leu Arg Ser Ala Gly
           115                 120                 125
   gag gtg gag atc cag ttc cgc cgc gtc aag tcc aag tac ccg gag ggc     432
   Glu Val Glu Ile Gln Phe Arg Arg Val Lys Ser Lys Tyr Pro Glu Gly
       130                 135                 140
   acc aag gtg acc ttc cac gtg gag aag ggg tcc aac ccc aac tac ctg     480
   Thr Lys Val Thr Phe His Val Glu Lys Gly Ser Asn Pro Asn Tyr Leu
   145                 150                 155                 160
   gcg ctg ctg gtg aag ttt gtc gcc ggc gac ggc gac gtg gtg gcg gtg     528
   Ala Leu Leu Val Lys Phe Val Ala Gly Asp Gly Asp Val Val Ala Val
                   165                 170                 175
   gac atc aag gag aag ggc aag gac aag tgg atc gcg ctc aag gag tcg     576
   Asp Ile Lys Glu Lys Gly Lys Asp Lys Trp Ile Ala Leu Lys Glu Ser
               180                 185                 190
   tgg gga gcc atc tgg agg atc gac acc ccg gag gtg ctc aag ggc ccc     624
   Trp Gly Ala Ile Trp Arg Ile Asp Thr Pro Glu Val Leu Lys Gly Pro
           195                 200                 205
   ttc acc gtc cgc ggc gag gcc aag gac gtc atc ccc gag ggc tgg aag      672
   Phe Thr Val Arg Gly Glu Ala Lys Asp Val Ile Pro Glu Gly Trp Lys
       210                 215                 220
   gcc gac acc gcc tac gag tcc aag tga                                  699
   Ala Asp Thr Ala Tyr Glu Ser Lys
   225                 230
<210> 16
   <211> 232
   <212> PRT
   <213> Phleum pratense
<400> 16
   Ile Pro Lys Val Pro Pro Gly Pro Asn Ile Thr Ala Thr Tyr Gly Asp
   1               5                   10                  15
   Lys Trp Leu Asp Ala Lys Ser Thr Trp Tyr Gly Lys Pro Thr Ala Ala
               20                  25                  30
   Gly Pro Lys Asp Asn Gly Gly Ala Ser Gly Tyr Lys Asp Val Asp Lys
           35                  40                  45
   Pro Pro Phe Ser Gly Met Thr Gly Ser Gly Asn Thr Pro Ile Phe Lys
       50                  55                  60
   Ser Gly Arg Gly Ser Gly Ser Ser Phe Glu Ile Lys Ser Thr Lys Pro
   65                  70                  75                  80
   Glu Ala Ser Ser Gly Glu Pro Val Val Val His Ile Thr Asp Asp Asn
                   85                  90                  95
   Glu Glu Pro Ile Ala Ala Tyr His Phe Asp Leu Ser Gly Ile Ala Phe
               100                 105                 110
   Gly Ser Met Ala Lys Lys Gly Asp Glu Gln Lys Leu Arg Ser Ala Gly
           115                 120                 125
   Glu Val Glu Ile Gln Phe Arg Arg Val Lys Ser Lys Tyr Pro Glu Gly
       130                 135                 140
   Thr Lys Val Thr Phe His Val Glu Lys Gly Ser Asn Pro Asn Tyr Leu
   145                 150                 155                 160
   Ala Leu Leu Val Lys Phe Val Ala Gly Asp Gly Asp Val Val Ala Val
                   165                 170                 175
   Asp Ile Lys Glu Lys Gly Lys Asp Lys Trp Ile Ala Leu Lys Glu Ser
               180                 185                 190
   Trp Gly Ala Ile Trp Arg Ile Asp Thr Pro Glu Val Leu Lys Gly Pro
           195                 200                 205
   Phe Thr Val Arg Gly Glu Ala Lys Asp Val Ile Pro Glu Gly Trp Lys
       210                 215                 220
   Ala Asp Thr Ala Tyr Glu Ser Lys
   225                 230
<210> 17
   <211> 666
   <212> DNA
   <213> Phleum pratense
<220>
   <221> CDS
   <222> (1)..(666)
   <223>
<400> 17
   ggc ccg aac atc acg gcg acc tac ggc gac aag tgg ctg gac gcg aag      48
   Gly Pro Asn Ile Thr Ala Thr Tyr Gly Asp Lys Trp Leu Asp Ala Lys
   1               5                   10                  15
   agc acc tgg tac ggc aag ccg acg gcc gcc ggt ccc aag gac aac ggc      96
   Ser Thr Trp Tyr Gly Lys Pro Thr Ala Ala Gly Pro Lys Asp Asn Gly
               20                  25                  30
   ggc gcg agc ggg tac aag gac gtg gac aag ccc ccg ttc agc ggc atg     144
   Gly Ala Ser Gly Tyr Lys Asp Val Asp Lys Pro Pro Phe Ser Gly Met
           35                  40                  45
   acc ggc tcc ggc aac acc ccc atc ttc aag tcc ggc cgg ggc agc ggc     192
   Thr Gly Ser Gly Asn Thr Pro Ile Phe Lys Ser Gly Arg Gly Ser Gly
       50                  55                  60
   tcc tcc ttc gag atc aag agc acc aag ccc gag gcc tcc tcc ggc gag     240
   Ser Ser Phe Glu Ile Lys Ser Thr Lys Pro Glu Ala Ser Ser Gly Glu
   65                  70                  75                  80
   ccc gtg gtg gtc cac atc acc gac gac aac gag gag ccc atc gcc gcg      288
   Pro Val Val Val His Ile Thr Asp Asp Asn Glu Glu Pro Ile Ala Ala
                   85                  90                  95
   tac cac ttc gac ctc tcc ggc atc gcg ttc ggg tcc gac gag cag aag      336
   Tyr His Phe Asp Leu Ser Gly Ile Ala Phe Gly Ser Asp Glu Gln Lys
               100                 105                 110
   ctg cgc agc gcc ggc gag gtg gag atc cag ttc cgc cgc gtc aag tcc      384
   Leu Arg Ser Ala Gly Glu Val Glu Ile Gln Phe Arg Arg Val Lys Ser
           115                 120                 125
   aag tac ccg gag ggc acc aag gtg acc ttc cac gtg gag aag ggg tcc      432
   Lys Tyr Pro Glu Gly Thr Lys Val Thr Phe His Val Glu Lys Gly Ser
       130                 135                 140
   aac ccc aac tac ctg gcg ctg ctg gtg aag ttt gtc gcc ggc gac ggc      480
   Asn Pro Asn Tyr Leu Ala Leu Leu Val Lys Phe Val Ala Gly Asp Gly
   145                 150                 155                 160
   gac gtg gtg gcg gtg gac atc aag gag aag ggc aag gac aag tgg atc      528
   Asp Val Val Ala Val Asp Ile Lys Glu Lys Gly Lys Asp Lys Trp Ile
                   165                 170                 175
   gcg ctc aag gag tcg tgg gga gcc atc tgg agg atc gac acc ccg gag      576
   Ala Leu Lys Glu Ser Trp Gly Ala Ile Trp Arg Ile Asp Thr Pro Glu
               180                 185                 190
   gtg ctc aag ggc ccc ttc acc gtc cgc ggc gag gcc aag gac gtc atc      624
   Val Leu Lys Gly Pro Phe Thr Val Arg Gly Glu Ala Lys Asp Val Ile
           195                 200                 205
   ccc gag ggc tgg aag gcc gac acc gcc tac gag tcc aag tga              666
   Pro Glu Gly Trp Lys Ala Asp Thr Ala Tyr Glu Ser Lys
       210                 215                 220
<210> 18
   <211> 221
   <212> PRT
   <213> Phleum pratense
<400> 18
   Gly Pro Asn Ile Thr Ala Thr Tyr Gly Asp Lys Trp Leu Asp Ala Lys
   1               5                   10                  15
   Ser Thr Trp Tyr Gly Lys Pro Thr Ala Ala Gly Pro Lys Asp Asn Gly
               20                  25                  30
   Gly Ala Ser Gly Tyr Lys Asp Val Asp Lys Pro Pro Phe Ser Gly Met
           35                  40                  45
   Thr Gly Ser Gly Asn Thr Pro Ile Phe Lys Ser Gly Arg Gly Ser Gly
       50                  55                  60
   Ser Ser Phe Glu Ile Lys Ser Thr Lys Pro Glu Ala Ser Ser Gly Glu
   65                  70                  75                  80
   Pro Val Val Val His Ile Thr Asp Asp Asn Glu Glu Pro Ile Ala Ala
                   85                  90                  95
   Tyr His Phe Asp Leu Ser Gly Ile Ala Phe Gly Ser Asp Glu Gln Lys
               100                 105                 110
   Leu Arg Ser Ala Gly Glu Val Glu Ile Gln Phe Arg Arg Val Lys Ser
           115                 120                 125
   Lys Tyr Pro Glu Gly Thr Lys Val Thr Phe His Val Glu Lys Gly Ser
       130                 135                 140
   Asn Pro Asn Tyr Leu Ala Leu Leu Val Lys Phe Val Ala Gly Asp Gly
   145                 150                 155                 160
   Asp Val Val Ala Val Asp Ile Lys Glu Lys Gly Lys Asp Lys Trp Ile
                   165                 170                 175
   Ala Leu Lys Glu Ser Trp Gly Ala Ile Trp Arg Ile Asp Thr Pro Glu
               180                 185                 190
   Val Leu Lys Gly Pro Phe Thr Val Arg Gly Glu Ala Lys Asp Val Ile
           195                 200                 205
   Pro Glu Gly Trp Lys Ala Asp Thr Ala Tyr Glu Ser Lys
       210                 215                 220
<210> 19
   <211> 18
   <212> DNA
   <213> Phleum pratense
<400> 19
   atcccgaagg tcccgccg 18
<210> 20
   <211> 63
   <212> DNA
   <213> Phleum pratense
<400> 20
   atcccgaagg tcccgccggg cccgaacatc acggcgacct acggcgacaa gtggctggac 60
   gcg 63
<210> 21
   <211> 63
   <212> DNA
   <213> Phleum pratense
<400> 21
   gttgtccttg ggaccggcgg ccgtcggctt gccgtaccag gtgctcttcg cgtccagcca 60
   ctt 63
<210> 22
   <211> 62
   <212> DNA
   <213> Phleum pratense
<400> 22
   ggtcccaagg acaacggcgg cgcgagcggg tacaaggacg tggacaagcc cccgttcagc 60
   gg 62
<210> 23
   <211> 69
   <212> DNA
   <213> Phleum pratense
<400> 23
   gagccgctgc cccggccgga cttgaagatg ggggtgttgc cggagccggt catgccgctg 60
   aacgggggc 69
<210> 24
   <211> 69
   <212> DNA
   <213> Phleum pratense
<400> 24
   tccggccggg gcagcggctc ctccttcgag atcaagagca ccaagcccga ggcctcctcc 60
   ggcgagccc 69
<210> 25
   <211> 30
   <212> DNA
   <213> Phleum pratense
<400> 25
   ggtaagcttt cacttggact cgtaggcggt 30
<210> 26
   <211> 23
   <212> DNA
   <213> Phleum pratense
<400> 26
   tccgggtact tggacttgac gcg 23
<210> 27
   <211> 23
   <212> DNA
   <213> Phleum pratense
<400> 27
   cgcgtcaagt ccaagtaccc gga 23
<210> 28
   <211> 42
   <212> DNA
   <213> Phleum pratense
<400> 28
   ccgaacatca cggcgaccta cggcgacaag tggctggacg cg 42
<210> 29
   <211> 69
   <212> DNA
   <213> Phleum pratense
<400> 29
   gtcgaagtgg tacgcggcga tgggctcctc gttgtcgtcg gtgatgtgga ccaccacggg 60
   ctcgccgga 69
<210> 30
   <211> 69
   <212> DNA
   <213> Phleum pratense
<400> 30
   gccgcgtacc acttcgacct ctccggcatc gcgttcgggt ccgacgagca gaagctgcgc 60
   agcgccggc 69
<210> 31
   <211> 96
   <212> DNA
   <213> Phleum pratense
<400> 31
   ggtaagcttt cacttggact cgtaggcggt gtcggccttc cagccctcgg ggatgacgtc     60
   cttggcctcg ccgcggacgg tgaaggggcc cttgag                               96

## Beschreibung

Die vorliegende Erfindung betrifft die Herstellung und Verwendung von Varianten von PhI p 1 aus dem Pollen von Wiesenlieschgras (*Phleum pratense*), welche durch eine gegenüber dem bekannten Wildtyp-Allergen verringerte IgE-Reaktivität und gleichzeitig eine weitgehend erhaltene Reaktivität mit T-Lymphozyten gekennzeichnet sind.
Diese hypoallergenen Allergenvarianten können zur spezifischen Immuntherapie (Hyposensibilisierung) von Patienten mit Gräserpollenallergie oder zur präventiven Immuntherapie von Gräserpollenallergien eingesetzt werden.

### Hintergrund der Erfindung

Allergien vom Typ 1 haben weltweite Bedeutung. Bis zu 20 % der Bevölkerung in industrialisierten Ländern leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40 % dieser Typ 1-Allergiker wiederum zeigen spezifische IgE-Reaktivität mit Gräserpollenallergenen (Freidhoff et al., 1986, J. Allergy Clin. Immunol. 78: 1190-2002).
Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Motekülen. Werden zwei IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden klinischen Symptomen.
In Abhängigkeit von der relativen Häufigkeit mit der die einzelnen Allergenmoleküle mit den IgE-Antikörpern von Allergikern reagieren, wird zwischen Major- und Minorallergenen unterschieden.
Im Fall von Wiesenlieschgras (*Phleum pratense*) sind bislang PhI p 1 (Petersen et al., 1993, J. Allergy Clin. Immunol. 92: 789-796), PhI p 5 (Matthiesen und Löwenstein, 1991, Clin. Exp. Allergy 21: 297-307; Petersen et al., 1992, Int. Arch. Allergy Immunol. 98: 105-109), PhI p 6 (Petersen et al., 1995, Int. Arch. Allergy Immunol. 108, 49-54). PhI p 2/3 (Dolecek et al., 1993, FEBS 335 (3), 299-304), PhI p 4 (Haavik et al., 1985, Int. Arch. Allergy Appl. Immunol. 78: 260-268; Valenta et al., 1992, Int. Arch. Allergy Immunol. 97: 287-294, Fischer et al., 1996, J. Allergy Clin. Immunol. 98: 189-198) und PhI p 13 (Suck et al., 2000, Clin. Exp. Allergy 30: 324-332; Suck et al., 2000, Clin. Exp. Allergy 30: 1395-1402) als Majorallergene identifiziert worden.

Die dominierenden Majorallergene des Wiesenlieschgrases (*Phleum pratense*) sind PhI p 1 und PhI p 5. Da die Majorallergene der Gräser der Familie *Poaceae* untereinander hoch homolog sind und infolge dessen sehr ähnliche biochemische und immunologische Eigenschaften besitzen, werden diese verwandten Proteine als Gruppe 1- bzw. Gruppe 5-Allergene zusammengefaßt.
Die Gruppe 1-Allergene reagieren mit den IgE-Antikörpern von mehr als 95 % der Gräserpollenallergiker und sind somit dominierende Majorallergene der Gräserpollen.
Die Gruppe 1-Allergene sind Glykoproteine mit einer Molekularmasse von ca. 32 kDa und sind im Zytoplasma der Pollenkörner lokalisiert. Sowohl der Kontakt der Pollenkörner mit der Schleimhaut der oberen Atmungswege als auch die Befeuchtung der Pollenkörner durch Regen führen zu einer raschen Freisetzung dieser Allergene. Die rasche Freisetzung der Gruppe 1-Allergene, auch in Form von subzellulären Mikropartikeln, ermöglicht das Eindringen in die unteren Luftwege, was zur Auslösung von schweren Asthmaanfällen führen kann.

Die cDNA's der Gruppe 1-Allergene von *Phleum pratense* (Laffer et al., 1994, J. Allergy Clin. Immunol. 94: 689-698), *Lolium perenne* (Perez et al., 1990, J. Biol. Chem. 265: 16210-16250), *Holcus lanatus* (Schramm et al., 1997, J. Allergy Clin. Immunol. 1999:781-787), *Poa pratensis* (Sturaro u. Viotti, 1998, NCBI GenBank, Acc. No. AJ 131850), *Cynodon dactylon* (Smith et al., 1996, J. Allergy Clin. Immunol. 98 : 331-343), *Phalaris aquatica* (Suphioglu et al., 1995, Clin. Exp. Allergy 25 : 853-865) und *Oryza sativa* (Xu et al., 1995, Gene 164: 255-259) sind identifiziert worden.

Außer diesen Erstbeschreibungen der Sequenz sind in Datenbanken weitere Gruppel-Allergen-Sequenzen publiziert, die in einzelnen Positionen von den originären Sequenzen abweichen. Solche Isoformen sind auch von anderen Gräserpollenallergenen bekannt.

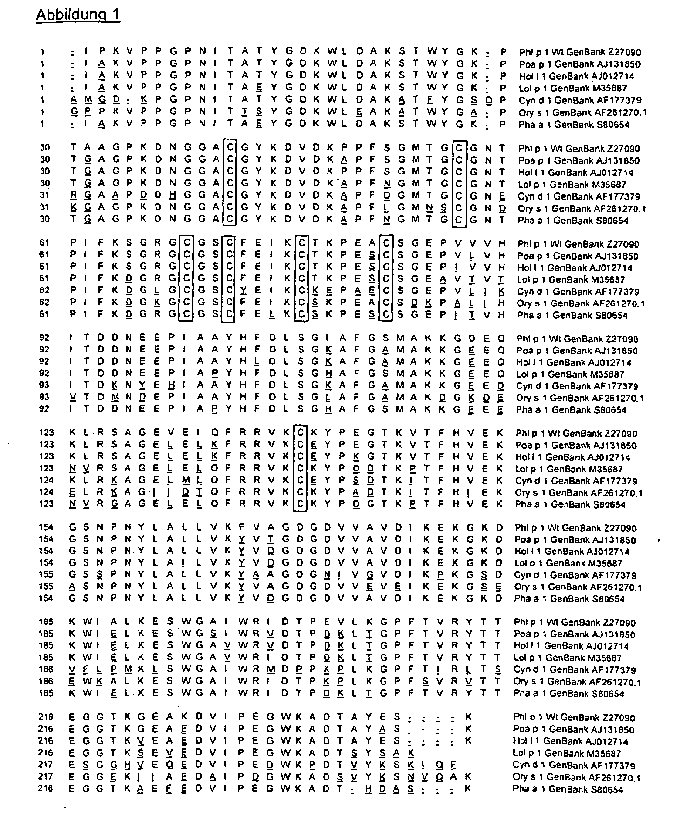

Die Gruppe 1-Allergene der Süßgräser (*Poaceae*) weisen aufgrund ihrer Homologie eine hohe Kreuzreaktivität mit humanen IgE-Antikörpern auf (Laffer et al., 1996, Mol. Immunology 33: 417-426). Diese immunologische Kreuzreaktivität basiert auf einer sehr ähnlichen Aminosäuresequenz, wie ein Sequenzvergleich von PhI p 1, dem Gruppe 1-Allergen des Wiesenlieschgrases (*Phleum pratense*), mit Gruppe 1-Molekülen von ausgewählten Spezies in Abbildung 1 zeigt.

Sowohl für die Sequenzbereiche der hier bei der Konstruktion hypoallergener Varianten beschriebenen Deletionen der PhI p 1-Aminosäuresequenz als auch für deren flankierenden Sequenzbereiche existieren homologe Sequenzbereiche in den übrigen Gruppe 1-Allergenen der *Poaceae.* Weiterhin sind sowohl die Anzahl als auch die umgebenden Sequenzbereiche der Cysteine der Gruppe 1-Allergene der *Poaceae* konserviert. Aufgrund von Sequenzhomologien werden die Gruppe 1-Allergene der *Poaceae* der Proteinfamile der β-Expansine zugeordnet (Cosgrove D.J., 2000 Nature 407: 321-6).

Ein klassischer Ansatz zur wirksamen therapeutischen Behandlung von Allergien stellt die spezifische Immuntherapie oder Hyposensibilisierung dar (Fiebig, 1995, Allergo J. 4 (6): 336-339: Bousquet et al., 1998, J. Allergy Clin. Immunol. 102 (4): 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen. Diese T-Zell-Epitope sind für die therapeutische Wirkung der Allergenpräparate bei der Hyposensibilisierung von entscheidender Bedeutung (Fiebig, 1995, Allergo J. 4 (7): 377-382).

Eine weitergehende Therapieoptimierung wäre mit rekombinant hergestellten Allergenen möglich. Definierte, ggf. auf die individuellen Sensibilierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.

Realistische Perspektiven, die zu einer sicheren Hyposensibilierung mit rekombinanten Expressionsprodukten führen können, bieten gezielt mutierte rekombinante Allergene, bei denen IgE-Epitope spezifisch deletiert werden, ohne die für die Therapie essentiellen T-Zell-Epitope zu beeinträchtigen (Schramm et al., 1999, J. Immunol. 162: 2406-2414).

Ein davon abweichendes Konzept für eine Hyposensibilisierung geht davon aus, dass eine protektive Immunantwort besonders durch blockierende IgG4-Antikörper induziert wird. Entsprechend dieser Hypothese sind rekombinante PhI p 1-Fragmente beschrieben worden, die zur Induktion einer protektiven IgG4-Antwort geeignet sein sollen (Ball et al.,1999, FASEB J. 13:1277-1290). Dieses Konzept unterscheidet sich vollständig von dem Konzept der hypoallergenen Allergenvarianten mit verringerter IgE-Reaktivität und erhaltener T-Zell-Reaktivität.

Eine weitere Möglichkeit zur therapeutischen Beeinflussung des gestörten T-Helferzellen-Gleichgewichtes bei Allergikern ist die Behandlung mit expressionsfähiger DNA, die für die relevanten Allergene kodiert (immuntherapeutische DNA-Vakzinierung). Experimentelle Belege für die allergenspezifische Beeinflussung der Immunantwort wurden an Nagem durch Injektion von Allergen-kodierender DNA erbracht (Hsu et al., 1996, Nature Medicine 2 (5): 540-544).

Die der vorliegenden Erfindung zugrunde liegende Aufgabe bestand nun in der Bereitstellung neuartiger Varianten von PhI p 1 auf Protein- und DNA-Ebene, die sich bei weitgehendem Erhalt der T-Zell-Reaktivität durch eine reduzierte IgE-Aktivität auszeichnen und daher für die kurative und präventive spezifische Immuntherapie sowie die immuntherapeutische DNA-Vakzinierung geeignet sind.

### Abbildungen

Abbildung 1: Alignment PhI p 1-homologer Aminosäuresequenzen (Sequenzen reifer Proteine deduziert aus cDNA-Sequenzen) von Spezies der Poaceae: Poa pratensis (Poa p), Holcus lanatus (Hol I), Lolium perenne (Lol p), Cynodon dactylon (Cyn d), Oryza sativa (Ory s) und Phalaris aquatica (Pha a), Proteinsequenzen deduziert von cDNA-Sequenzen der "GenBank"-Datenbank des National Center for Biotechnology Information (NCBI, Bethesda, USA), Nummerierung: Aminosäurepositionen reifer Proteine, Durch Unterstreichung gekennzeichnet: Zu PhI p 1-Sequenz differente Aminosäuren, Schwarzer Rahmen: Cysteine

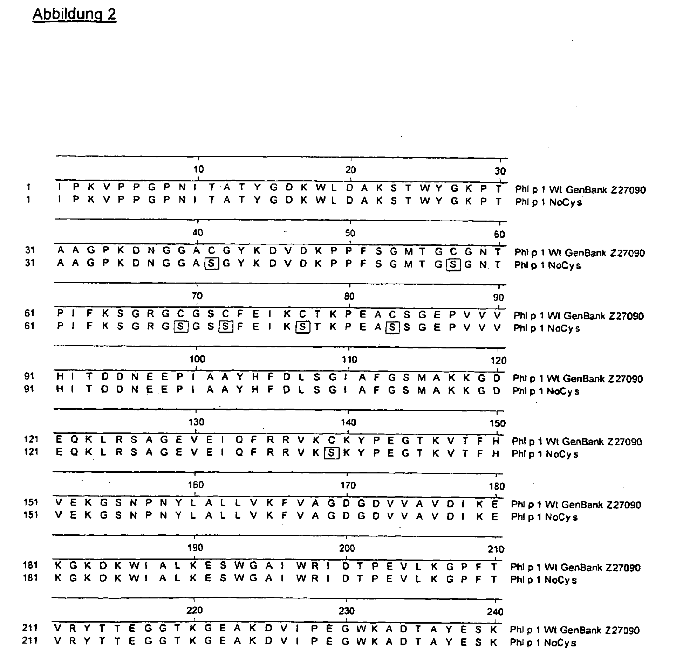

Abbildung 2: Alignment von Aminosäuresequenzen des prozessierten PhI p1 Wildtyp-Proteins und der Variante PhI p1 NoCys,
PhI p1 Wt (Wildtyp): Proteinsequenz deduziert aus cDNA-Sequenz ("GenBank"-Datenbankeintrag Z27090 des National Center for Biotechnology Information (NCBI), Bethesda, USA), Nummerierung: Aminosäurepositionen des reifen Proteins, Schwarz eingerahmt: Aminosäuresubstitutionen Cystein gegen Serin in Protein PhI p 1 NoCys

Abbildung 3: Alignment von Aminosäuresequenzen der beispielhaft dargestellten hypoallergenen Varianten PhI p 1 NoCys, PhI p 1 NoCys Δ213-220 und PhI p 1 NoCys Δ1-6, 115-119, 213-220, Nummerierung: Aminosäurepositionen, Durch Unterstreichung gekennzeichnet: Deletionen

Abbildung 4: SDS-PAGE und Identitätsüberprüfung der rekombinanten Varianten PhI p 1 NoCys, PhI p 1 NoCys Δ213-220 und PhI p 1 NoCys Δ1-6, 115-119, 213-220
A: SDS-PAGE
B: Western Blot mit aPhI p 1-Antikörper (Allergopharma)
   1. Markerproteine
   2. nPhI p 1*
   3. rPhI p 1 Wt (- His-tag)*
   4. Markerproteine
   5. PhI p 1 NoCys (+ His-tag)
   6. PhI p 1 NoCys D213-220 (+ His-tag)
   7. PhI p 1 NoCys D1-6, 115-119, 213-220 (+ His-tag)
   8. Markerproteine
      * Proben reduziert (Dithiotreitol)

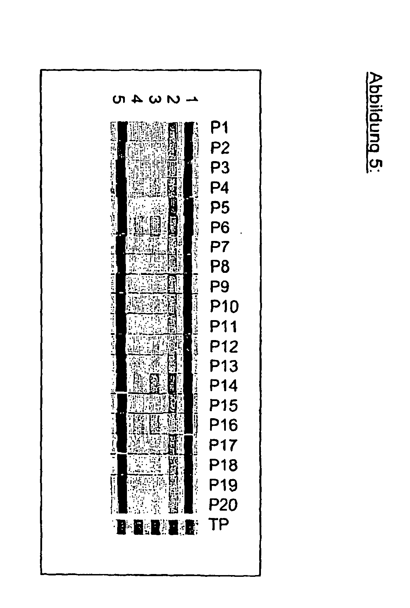

Abbildung 5: Streifentest zur Überprüfung der IgE-Bindungsfähigkeit von PhI p 1 NoCys, PhI p 1 NoCys Δ213-220 und PhI p 1 NoCys Δ1-6, 115-119, 213-220 unter nicht-denaturierenden Bedingungen
1) rPhI p 1 Wt
2) PhI p 1 NoCys
3) PhI p 1 NoCys D213-220
4) PhI p 1 NoCys D1-6, 115-119, 213-220
5) rPhI p 1 Wt
TP: Gesamtproteinfärbung
P: Seren klinisch definierter Gräserpollen-Allergiker

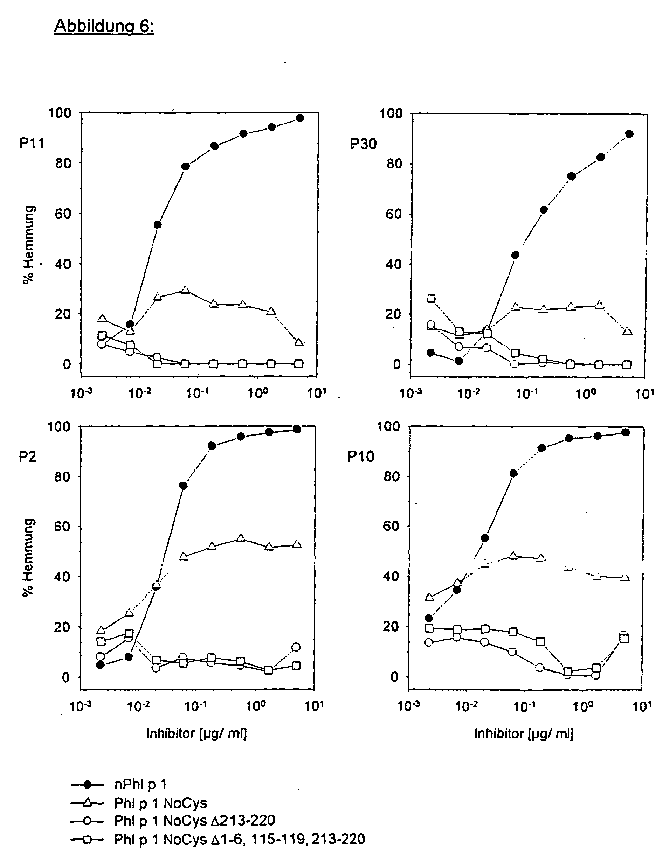

Abbildung 6: Nachweis der reduzierten IgE-Reaktivität von PhI p 1 NoCys, PhI p 1 NoCys Δ213-220 und PhI p 1 NoCys Δ1-6, 115-119, 213-220 mittels EAST-Inhibitionstest mit vier repräsentativen Seren von Gräserpollen-Allergikern (P)

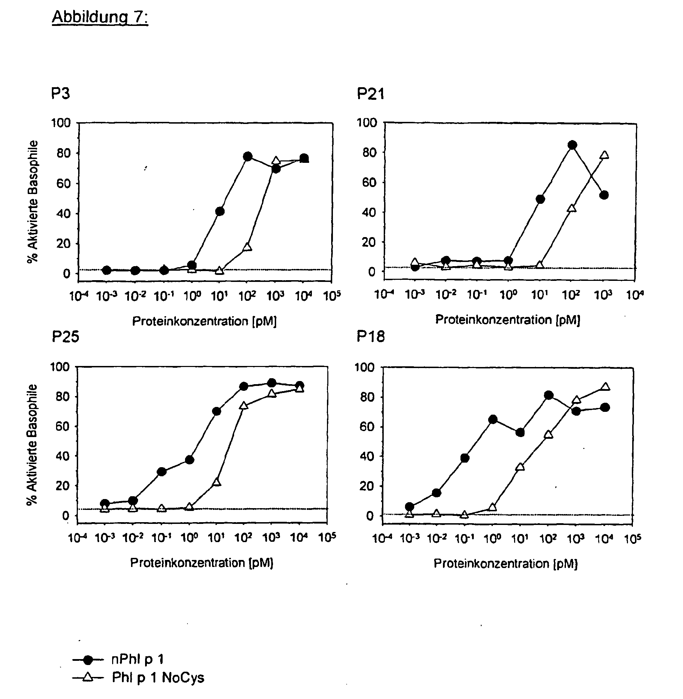

Abbildung 7: Nachweis der Hypoallergenität von PhI p 1 NoCys mittels Basophilen-Aktivierungstest mit Basophilen von vier Gräserpollen-Allergikern (P)

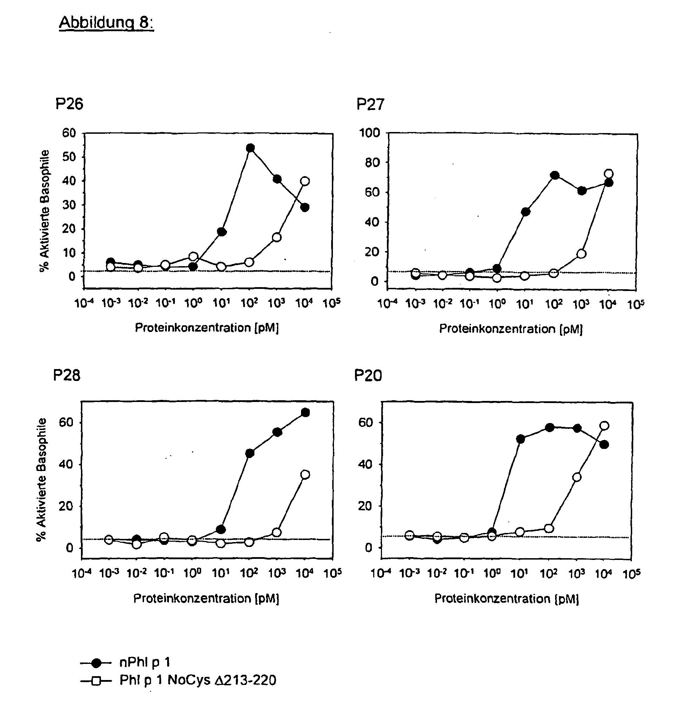

Abbildung 8: Nachweis der Hypoallergenität von PhI p 1 NoCys Δ213-220 mittels Basophilen-Aktivierungstest mit Basophilen von vier Gräserpollen-Allergikern (P)

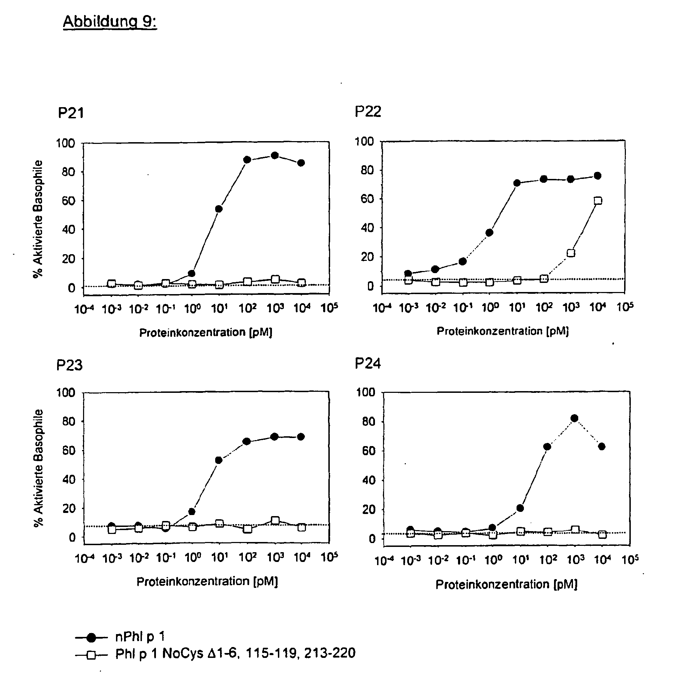

Abbildung 9: Nachweis der Hypoallergenität von PhI p 1 NoCys Δ1-6, 115-119, 213-220 mittels Basophilen-Aktivierungstest mit Basophilen von vier Gräserpollen-Allergikern (P)

Die Arbeiten, die zu den aufgefundenen Varianten führten, wurden anhand von PhI p 1 durchgeführt.

Gegenstand der vorliegenden Erfindung sind somit Varianten von PhI p 1, welche durch eine gegenüber den bekannten Wildtyp-Allergenen verringerte IgE-Reaktivität sowie eine erhaltene Reaktivität mit T-Lymphozyten gekennzeichnet sind.

Ausgangspunkt für die Konstruktion der hypoallergenen Varianten von PhI p 1_ist die cDNA des PhI p 1 Wildtyps, die mit Hilfe spezifischer Primer durch Polymerase-Kettenreaktion (PCR) aus der Gesamt-cDNA von Pollen von *Phleum pratense* isoliert wurde ("GenBank" Eintrag Z27090; NCBI, Bethesda, USA) (SEQ ID NO 1).
Von der cDNA-Sequenz des PhI p 1 Wildtyps wurde die Aminosäuresequenz gemäß SEQ ID NO 2 deduziert.
Das aus 240 Aminosäuren bestehende, in der natürlichen Form glykosylierte PhI p 1 ist - wie alle Gruppe 1-Allergene (siehe Abb. 1) - durch die Existenz von sieben Cysteinen des reifen Moleküls gekennzeichnet. Mit der Ausnahme von Cyn d 1 und Ory s1 haben diese Aminosäurepositionen bei allen Gruppe 1-Allergenen die Nummern 41, 57, 69, 72, 77, 83 und 139 (Petersen et al., 1995, J. Allergy Clin. Immunol 95: 987-994).

PhI p 1 konnte in *E*. *coli* als nicht-glykosyliertes Protein exprimiert werden. Das rekombinante Wildtyp-Protein (rPhI p 1 wt) reagierte mit IgE-Antikörpern von Gräserpollenallergikern, die eine Reaktivität mit natürlichem gereinigten PhI p 1 (nPhI p 1) aufwiesen (Petersen et al., 1998, Clin. Exp. Allergy 28: 315-321).

### Detaillierte Beschreibung der Erfindung

### Herstellung und Charakterisierung hypoallergener PhI p 1-Varianten

Ausgehend von der beschriebenen cDNA von rPhI p 1 wt wurden gentechnisch veränderte rekombinante Varianten von PhI p 1 hergestellt.

Die Aminosäuresequenz der rekombinanten Variante PhI p 1 NoCys (SEQ ID NO 4) weist anstelle der im Wildtyp vorkommenden sieben Cysteine sieben Serinreste auf (Abb. 2). Die Variante PhI p 1 NoCys diente als Ausgangspunkt für die Konstruktion von unterschiedlichen Deletionsmutanten. Bei diesen sind jeweils einzelne Abschnitte von 15 bis 90 bp Länge oder Kombinationen dieser Abschnitte der cDNA kodierend für PhI p 1 NoCys deletiert, wodurch entsprechende Deletionen der Aminosäuren 1-6, 1-30, 92-104, 115-119, 175-185 und 213-220 in den Polypeptidketten der in *E*. *coli* exprimierten Proteine resultieren: PhI p 1 NoCys Δ1-6 (SEQ ID NO 5 und 6), PhI p 1 NoCys Δ1-30 (SEQ ID NO 7 und 8), PhI p 1 NoCys Δ92-104 (SEQ ID NO 9 und 10), PhI p 1 NoCys Δ115-119 (SEQ ID NO 11 und 12), PhI p 1 NoCys Δ175-185 (SEQ ID NO 13 und 14), PhI p 1 NoCys Δ213-220 (SEQ ID NO 15 und 16), sowie PhI p 1 NoCys Δ1-6, 115-119, 213-220 (SEQ ID NO 17 und 18).

Die Expression der rekombinanten Proteine erfolgte als Histidin-Fusionsproteine in *Escherichia coli.* Die immunologische Charakterisierung wurde mit solchen Fustionspoteinen durchgeführt.

Zunächst wurden die rekombinanten Varianten nach Immobilisierung an eine Nitrocellulose-Membran auf die Fähigkeit, von IgE-Antikörpern eines repräsentativen Serum pools und von IgE-Antikörpern von individuellen Seren von Gräserpollenallergikem gebunden zu werden, untersucht (Streifentest). Bei diesem Verfahren wurde überraschenderweise eine verminderte Bindung von IgE-Antikörpern an PhI p 1 NoCys beobachtet. Dieses Ergebnis wurde durch einen IgE-Inhibitionstest (EAST), bei dem die IgE-Bindungsfähigkeit eines nicht-immobilisierten Proteins an IgE-Antikörper in Lösung untersucht wird, bestätigt.

Gegenstand der vorliegenden Erfindung sind daher insbesondere solche Gruppe 1-Allergenvarianten, bei denen die Cysteine der dem reifen PhI p 1 Protein entsprechenden Aminosäurepositionen 41, 57, 69, 72, 77, 83 und 139 entfernt oder gegen eine andere Aminosäure ersetzt werden.

Eine verminderte Bindung von IgE-Antikörpern wird bereits dann erhalten, wenn mindestens zwei der 7 Cysteine ersatzlos entfernt oder gegen eine andere Aminosäure ersetzt werden. Vorzugsweise werden jedoch alle 7 Cysteine gegen Serin ausgetauscht.

Mittels eines Aktivierungstests von basophilen Granulozyten von Gräserpollenallergikern wurden die Auswirkungen der reduzierten IgE-Bindungsfähigkeit von PhI p 1 NoCys auf die funktionelle Wirkung bei der Vernetzung von membrangebundenem IgE der Effektorzellen und deren Aktivierung *in vitro* untersucht. PhI p 1 NoCys zeigte hier eine deutlich geringere Aktivierung von basophilen Granulozyten im Vergleich zu rPhI p 1 wt und somit eine funktionell reduzierte Allergenität.

Die auf Basis von PhI p 1 NoCys hergestellten unterschiedlichen Deletionsmutanten wurden nach dem gleichen Verfahren hinsichtlich der IgE-Bindungsfähigkeit (Streifentest, EAST) und der funktionellen Wirkung (Basophilenaktivierung) untersucht. Überraschenderweise zeigten die Deletionsmutanten besonders starke hypoallergene Eigenschaften.

Gegenstand der vorliegenden Erfindung sind daher weiterhin solche PhI p 1-Allergenvarianten, bei denen - wahlweise zusätzlich zu den zuvor beschriebenen Varianten mit entferntem bzw. ersetztem Cys - mindestens ein Bereich oder eine Kombination von Bereichen, die den Aminosäuren 1-6, 1-30, 92-104, 115-119, 175-185 und 213-220 der Primärsequenz des reifen PhI p 1 Proteins entsprechen, gegenüber dem Wildtyp-Allergen fehlen.

Insbesondere bevorzugt und daher ebenfalls Erfindungsgegenstand sind diejenigen PhI p 1-Allergenvarianten, bei denen ausschließlich die Aminosäuren 213-220 bzw. gleichzeitig die Aminosäuren 1-6 und 115-119 entsprechend der reifen PhI p 1-Sequenz fehlen.

Die der Wirksamkeit der spezifischen Immuntherapie zugrundeliegende T-Zell-Reaktivität der hypoallergenen PhI p 1-Varianten wurde durch einen Proliferationstest mit PhI p 1-spezifischen T-Lymphozyten von Gräserpollenallergikern *in vitro* überprüft. Die modifizierten Allergene zeigten eine weitgehend erhaltene T-Zell-Reaktivität, was eine immuntherapeutische Verwendung der hypoallergenen PhI p 1-Varianten ermöglicht.

Es bietet sich an, die erfindungsgemäßen Allergenvarianten, ausgehend von der klonierten DNA-Sequenz mit Hilfe gentechnischer Methoden herzustellen. Grundsätzlich kann es sich aber auch um chemische Modifikationen des nativen Allergenextrakts handeln (Fiebig, 1995, Allergo J. 4 (7), 377-382).
Naturgemäß sind über die in der vorliegenden Patentanmeldung beschriebenen Variationen von Gruppe-1-Allergenen auch weitere Veränderungen an anderen Positionen - etwa zur Erhöhung der Hypoallergenität - möglich. Bei diesen Modifikationen kann es sich beispielsweise um Aminosäure-Insertionen, -Deletionen und -Austausche, Aufspaltungen des Proteins in Fragmente sowie Fusionen des Proteins oder seiner Fragmente mit anderen Proteinen oder Peptiden handeln.
Gegenstand ist weiterhin ein DNA-Molekül, kodierend für eine zuvor beschriebene Allergenvariante, ein rekombinanter Expressionsvektor, enthaltend dieses DNA-Molekül sowie ein Wirtsorganismus, transformiert mit besagtem DNA-Molekül oder besagtem Expressionsvektor. Geeignete Wirtsorganismen können pro- oder eukaryontische, ein- oder mehrzellige Organismen wie Bakterien oder Hefen sein. Ein erfindungsgemäß bevorzugter Wirtsorganismus ist *E*. *coli.*

Weiterhin betrifft die Erfindung ein Verfahren zur Herstellung einer erfindungsgemäßen Allergenvariante durch Kultivieren des besagten Wirtsorganismus und Gewinnung der entsprechenden Allergenvariante aus der Kultur.

Gegenstand der vorliegenden Erfindung sind außerdem die zuvor beschriebenen Allergenvarianten, DNA-Moleküle und Expressionsvektoren in ihrer Eigenschaft als Arzneimittel.

Weiterhin sind Gegenstand der vorliegenden Erfindung pharmazeutische Zubereitungen enthaltend mindestens eine dieser Allergenvarianten bzw. ein entsprechendes DNA-Molekül oder einen entsprechenden Expressionsvektor sowie gegebenenfalls weitere Wirk- und/oder Hilfsstoffe zur Behandlung von Allergien, an deren Auslösung Gruppe-1-Allergene der *Poaceae* beteiligt sind, bzw. zur immuntherapeutischen Vakzinierung von Patienten mit Allergien, an deren Auslösung Gruppe-1-Allergene der *Poaceae* beteiligt sind und/oder zur Prävention solcher Allergien.
Handelt es sich um pharmazeutische Zubereitungen des zweiten Typs (enthaltend mindestens ein DNA-Molekül oder einen Expressionsvektor), so enthalten diese Zubereitungen vorzugsweise weiterhin Aluminiumhydroxid, ein immunstimulatorisches CpG-haltiges Oligonukleotid oder eine Kombination beider als Hilfsstoffe.

Pharmazeutische Zubereitungen im Sinne dieser Erfindung können als Therapeutika in der Human- oder Veterinärmedizin verwendet werden. Als Trägerstoffe kommen organische oder anorganische Substanzen in Frage, die sich für die parenterale Applikation eignen und mit erfindungsgemäßen Gruppe-1-Allergenvarianten nicht reagieren. Zur parenteralen Anwendung dienen insbesondere Lösungen, vorzugsweise ölige oder wässrige Lösungen, ferner Suspensionen, Emulsionen oder Implantate. Die erfindungsgemäßen Allergenvarianten können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten. Weiterhin können durch entsprechende Formulierung der erfindungsgemäßen Allergenvarianten Depotpräparate, beispielsweise durch Adsorption an Aluminiumhydroxid, erhalten werden.

Schließlich ist Gegenstand der vorliegenden Erfindung die Verwendung mindestens einer erfindungsgemäßen Allergenvariante bzw. eines erfindungsgemäßen DNA-Moleküls oder eines erfindungsgemäßen Expressionsvektors zur Herstellung eines Arzneimittels zur Behandlung von Allergien, an deren Auslösung Gruppe-1-Allergene der *Poaceae* beteiligt sind bzw. zur immuntherapeutischen Vakzinierung von Patienten mit Allergien, an deren Auslösung Gruppe-1-Allergene der *Poaceae* beteiligt sind und/oder zur Prävention solcher Allergien.

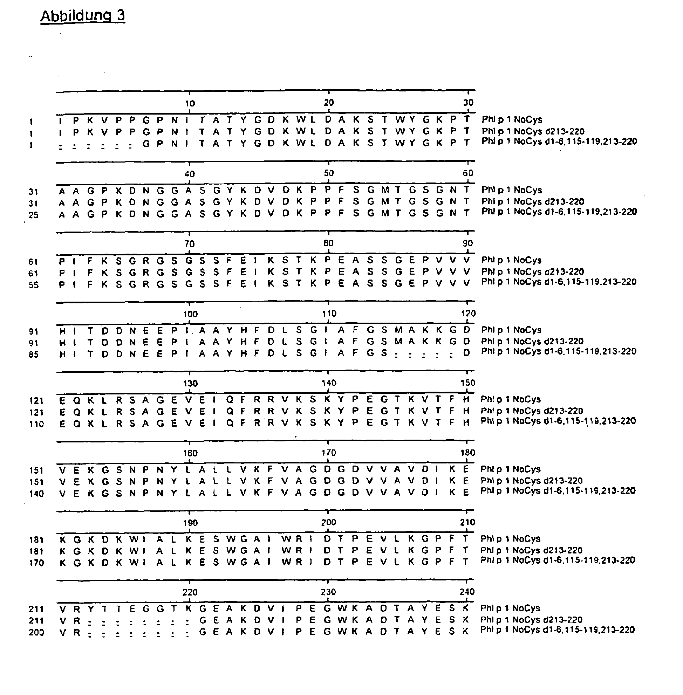

Die Herstellung der Varianten PhI p 1 NoCys, PhI p 1 NoCys Δ213-220 und PhI p 1 NoCys Δ1-6, 115-119, 213-220 (Abb. 3) sowie deren immunologische Charakterisierung ist im folgenden beispielhaft für die hypoallergenen PhI p 1-Varianten mit den oben beschriebenen gentechnischen Modifikationen dargestellt.

### Expression und Reinigung rekombinanter PhI p 1-Varianten

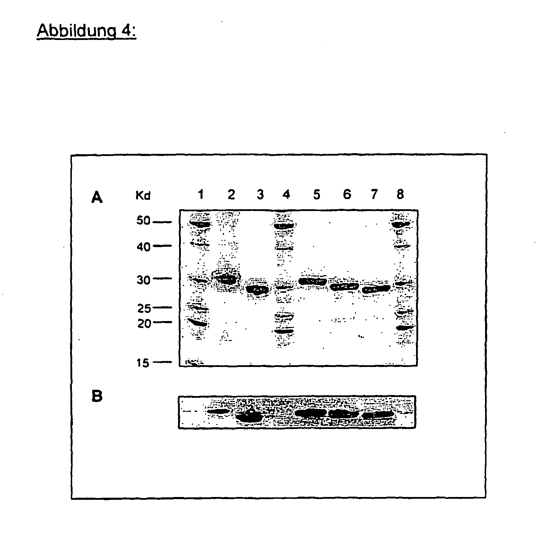

Die Expression der rekombinanten Proteine erfolgte als Histidin-Fusionsproteine (Expressionsvektor pProExHT; Invitrogen, Carlsbad, USA) in *Escherichia coli* (Stamm JM109). rPhI p 1 wt sowie die Varianten wurden zunächst durch die spezifische Bindung der N-terminalen Histidinreste an eine Ni²+-Chelat-Matrix (Immobilized-Metal-lon-Affinity-Chromatography, IMAC) und nachfolgend durch präparative Gelfiltration (Size-Exclusion-Chromatography, SEC) gereinigt. Die Reinheit der eluierten Proteine wurde durch SDS-PAGE und analytische SEC kontrolliert (Abb. 4a). Die Identität der gereinigten Proteine wurde durch die Bindung eines PhI p 1-spezifischen monoklonalen Antikörpers nachgewiesen (Abb. 4b).

### Nachweis der reduzierten IgE-Bindung der rekombinanten PhI p 1-Varianten

Eine einfache Testmethode zur Bestimmung der IgE-Reaktivität von spezifischem IgE aus Allergikerseren an membrangebundene Testproteine ist der Streifentest.
Dafür werden die Testsubstanzen in gleicher Konzentration und Menge nebeneinander an einen Streifen von Nitrocellulose-Membran unter nicht-denaturierenden Bedingungen gebunden. Eine Reihe solcher Membranstreifen kann parallel mit unterschiedlichen Allergikerseren inkubiert werden. Nach einem Waschschritt werden die spezifisch gebundenen IgE-Antikörper durch eine Farbreaktion, vermittelt von einem Anti-human IgE/Alkalische Phosphatase-Konjugat, auf der Membran sichtbar gemacht.

Beispielhaft für die beschriebenen modifizierten PhI p 1-Moleküle werden hier die Ergebnisse des Streifentests bezüglich PhI p 1 NoCys, PhI p 1 NoCys Δ213-220 und PhI p 1 NoCys Δ1-6, 115-119, 213-220 unter Einsatz von individuellen Gräserpollen-Allergikerseren dargestellt (Abb. 5). Es kamen nur Seren von Allergikern mit einem starken IgE-Titer gegen natürliches PhI p 1 zur Anwendung. Die IgE-Antikörper dieser Patienten reagieren ebenso mit dem rekombinanten Äquivalent rPhI p 1 wt.

Es wird deutlich, dass die PhI p 1-spezifischen IgE-Antikörper aller Patientenseren die rekombinante Variante PhI p 1 NoCys vermindert binden, nicht aber das Wildtypallergen PhI p 1.

Eine noch stärkere Reduktion der IgE-Bindungsfähigkeit wird durch das zusätzliche Entfernen bestimmter Sequenzabschnitte erzielt, was anhand an der Variante PhI p 1 NoCys Δ213-220 dargestellt ist. Die Variante PhI p 1 NoCys Δ213-220 zeigt mit allen überprüften Allergikerseren eine sehr stark verminderte IgE-Bindungsfähigkeit gegenüber dem nichtveränderten rekombinanten Wildtyp-Protein. Eine weitere Reduktion der IgE-Bindungsfähigkeit von PhI p 1 an IgE-Antikörper bestimmter Seren kann durch Kombination mehrerer Deletionen erzielt werden, was an dem Testergebnis der Variante PhI p 1 NoCys Δ1-6, 115-119, 213-220 mit dem Gräserpollen-Allergikerserum P14 erkennbar ist (Abb. 5).
Somit wird deutlich, dass sowohl die Substitution von Cysteinen als auch die Deletion von spezifischen Sequenzabschnitten die IgE-Bindungsfähigkeit des PhI p 1-Moleküls herabsetzt.

Im Gegensatz zu dem Streifentest können mit dem EAST-Hemmtest (Enzyme Allergosorbent Test) Allergen/ IgE-Interaktionen in Lösung untersucht werden, womit eine störende Maskierung von Epitopen der Testsubstanz durch die Immobilisierung an die Membran grundsätzlich ausgeschlossen werden kann.
Der EAST-Hemmtest wird wie folgt ausgeführt. Mikrotiterplatten werden mit Allergenen, hier nPhI p 1, beschichtet. Nach Entfernung der nicht gebundenen Allergenmoleküle durch Waschen wird die Platte zur Vermeidungspäterer unspezifischer Bindungen mit Rinderserumalbumin blockiert. IgE-Antikörper von Allergikern, als repräsentativer Pool von Einzelseren (Serum-Pool) oder als Einzelserum, wird in geeigneter Verdünnung mit den Allergen-beschichteten Mikrotiterplatten inkubiert. Die Menge der Allergengebundenen IgE-Antikörper wird über ein Anti-hlgE/ Alkalische Phosphatase-Konjugat durch die Umsetzung eines Substrates zu einem farbigen Endprodukt photometrisch quantifiziert.

Die Bindung der IgE-Antikörper wird durch ein lösliches Allergen bzw. die zu prüfende Substanz (rekombinantes modifiziertes Allergen) in Abhängigkeit von der Konzentration substanzspezifisch gehemmt.

Beispielhaft für die beschriebenen modifizierten PhI p 1-Moleküle werden hier die Testergebnisse bezüglich PhI p 1 NoCys, PhI p 1 NoCys Δ213-220 und PhI p 1 NoCys Δ1-6, 115-119, 213-220 im Vergleich zu dem Referenzmolekül nPhI p 1 dargestellt.

Die in Abbildung 6 dargestellten repräsentativen IgE-Inhibitionstests mit vier Individualseren von Gräserpollen-Allergikern zeigen, dass selbst mit hohen Konzentrationen der Variante PhI p 1 NoCys (bis 5 µg/ ml) nur ca. 20-50% der maximalen Hemmwirkung des unveränderten natürlichen Allergens nPhI p1 erzielt wurde. Die niedrigere maximale Hemmwirkung deutet auf einen Verlust von IgE-Epitopen hin.
Die Kurvenverläufe der Varianten PhI p 1 NoCys Δ213-220 und PhI p 1 NoCys Δ1-6, 115-119, 213-220 zeigen eine noch geringere IgE-Bindungsfähigkeit dieser PhI p 1-Varianten auf. Eine Hemmwirkung konnte individuell variierend nicht oder nur in sehr geringer Stärke (0-20% der maximalen Hemmwirkung) nachgewiesen werden.
Übereinstimmend mit dem Ergebnis des Streifentests kann somit belegt werden, dass durch Einfügen zusätzlicher gezielter Deletionen die IgE-Bindungsfähigkeit von PhI p 1 weiter reduziert wird.

### Nachweis der Hypoallergenität der rekombinanten PhI p 1-Varianten durch Basophilen-Aktivierungstest

Die funktionelle Reduktion der Allergenität wurde mittels eines Basophilenaktivierungstests *in vitro* bestimmt. Für den Basophilenaktivierungstest wird heparinisiertes Vollblut von Gräserpollen-Allergikern mit verschiedenen Konzentrationen der Testsubstanzen inkubiert. Die allergenen Substanzen werden von den FcεRI-gebundenen IgE-Antikörpern der Basophilen spezifisch gebunden und führen zu einer Vernetzung der FcεRI-Moleküle. Diese Allergen-induzierte IgE-vermittelte FcεRI-Vernetzung führt zur Aktivierung der Basophilen. Die Aktivierung ist der erste Schritt in der allergischen Reaktion dieser Effektorzellen. Die anschließende Signaltransduktion resultiert in der Degranulation der Effektorzellen und somit dem Auslösen der allergischen Reaktionen *in vivo.*

*In vitro* kann die Allergen-induzierte Aktivierung von basophilen Granulozyten durch Quantifizierung der Expression eines mit der Signaltransduktion der IgE-Rezeptor-Vernetzung gekoppelten Oberflächenproteins (CD203c) nachgewiesen werden (Kahlert et al., 2003, Cli., Exp. Allergy 33: 1266-72). Die Zahl der exprimierten CD203c-Proteine auf einer Zelle und der Prozentwert der aktivierten Zellen eines Zellpools wird über die Bindung eines fluoreszenzmarkierten monoklonalen Antikörpers an den Oberflächenmarker und anschließende Analyse durch fluoreszenzaktivierte Durchflusszytometrie hochsensitiv gemessen.
Als Referenzsubstanzen wurde hier das gereinigte natürliche PhI p 1 (nPhI p 1) parallel zu den Testsubstanzen eingesetzt. Beispielhaft für die beschriebenen modifizierten PhI p 1-Moleküle werden hier die Ergebnisse PhI p 1 NoCys, PhI p 1 NoCys Δ213-220 und PhI p 1 NoCys Δ1-6, 115-119, 213-220 dargestellt.

Repräsentative Testergebnisse der Variante PhI p 1 NoCys mit Basophilen von vier klinisch definierten Allergikern sind in Abbildung 7 als Kurvenverläufe gezeigt. Die Reduktion der allergenen Wirksamkeit der Variante PhI p 1 NoCys relativ zu nPhI p 1 Wildtyp wird durch die Verschiebung der Aktivierungskurven deutlich.
Die Testergebnisse der Varianten PhI p 1 NoCys Δ213-220 und PhI p 1 NoCys Δ1-6, 115-119, 213-220 weisen im Einklang mit den Ergebnissen des Streifentests und des IgE-Inhibitionstests auf eine noch stärkere Reduktion der relativen allergenen Wirksamkeit hin, wie in Abbildung 8 und 9 anhand repräsentativer Kurvenläufe gezeigt wird.
Während in einem Konzentrationsbereich der Testsubstanzen von 100-1000 pM durch das natürliche Allergen bereits ein maximaler Anteil von Basophilen aktiviert wurde, konnte keine oder eine nur sehr geringe Basophilen-Aktivierung bei Einsatz der modifizierten Allergene PhI p 1 NoCys Δ213-220 und PhI p 1 NoCys Δ1-6, 115-119, 213-220 nachgewiesen werden.
Die allergene Wirksamkeit der Variante PhI p 1 NoCys Δ213-220 war, wie über die A50-Werte der Kurvenverläufe berechnet werden kann, ∼ 100-1000 fach und die der Variante PhI p 1 NoCys Δ1-6, 115-119, 213-220 mehr als 1000 fach gegenüber der Referenz nPhI p 1 erniedrigt (A50: Allergenkonzentration bei 50% der Zahl maximal aktivierter Basophilen).

### T-Zell-Reaktivität der hypoallergenen PhI p 1-Varianten

T-Helfer-Lymphozyten reagieren mit Peptidfragmenten der Allergene (ca. 12-25 Aminosäuren), die durch enzymatischen Abbau in Antigenpräsentierenden Zellen (APZ) entstehen und nach Einlagerung der geeigneten Peptide in die individuellen MHC-Klasse II-Moleküle an der Oberfläche der APZ den T-Zellen präsentiert werden. Diese allergenspezifische Aktivierung der T-Helfer-Lymphozyten ist die Voraussetzung für Proliferation und für die funktionelle Differenzierung (TH1 und TH2). Die Beeinflussung der allergenspezifischen T-Lymphozyten durch die Behandlung mit Allergen oder einem Allergenderivat bei der Hyposensibilisierung wird als Schlüssel für die therapeutische Wirksamkeit angesehen.

Zur Untersuchung der T-Zell-Reaktivität werden oligoklonale T-Zell-Linien von Graspollen-Allergikern unter Stimulation mit nPhI p 1 - oder rPhI p 1 wt -Molekülen nach üblichen Verfahren etabliert. In einem Proliferationstest wurden die unterschiedlichen T-Zell-Linien mit den Referenz-Allergenen nPhI p 1 und rPhI p 1 wt sowie den modifizierten rekombinanten PhI p 1-Varianten stimuliert. Die Proliferationsrate wurde durch den Einbau von [³H]-Thymidin mit den üblichen Verfahren bestimmt.

Beispielhaft für die beschriebenen modifizierten PhI p 1-Moleküle sind hier die Ergebnisse des Proliferationstest von PhI p 1 NoCys, PhI p 1 NoCys Δ213-220 und PhI p 1 NoCys Δ1-6, 115-119, 213-220 dargestellt.

Die in Tabelle 1 dargestellten Ergebnisse mit T-Zell-Linien von acht Gräserpollen-Allergikern zeigen, dass die T-Lymphozyten durch die rekombinanten Allergenvarianten zur Proliferation stimuliert werden konnten. Die T-Zellreaktivität von PhI p 1 NoCys, PhI p 1 NoCys Δ213-220 und PhI p 1 NoCys Δ1-6, 115-119, 213-220 ist im Vergleich zu den unveränderten natürlichen und rekombinanten Wildtyp-Allergenen nur wenig erniedrigt, was die Erhaltung der entscheidenden T-Zell-Epitope nachweist.

**Tabelle 1: Nachweis der T-Zellreaktivität von PhI p 1 NoCys, PhI p 1 NoCys Δ213-220 und PhI p 1 NoCys Δ1-6, 115-119, 213-220 mittels Proliferationstests mit PhI p 1-reaktiven T-Zell-Linien (TCL)**

| | | Stimulationsindex¹ | | | | |
|---|---|---|---|---|---|---|
| Dono² | TCL | | rPhI p 1 Wt | PhI p 1 NoCys | PhI p 1 NoCys Δ213-220 | PhI p 1 NoCys Δ1-6, 115-119, 213-220 |
| A | 11.16 | 3,2 | 4,7 | 3,7 | 3,5 | 3,9 |
| B | 21.1 | 23,5 | 13,9 | 12,1 | 13,0 | 12,3 |
| C | 60.51 | 3,9 | 5,7 | 3,9 | 4,2 | 2,0 |
| D | 104.8 | 2,6 | 4,6 | 4,1 | 3,4 | 3,0 |
| E | 10.27 | 26,4 | 27,8 | 32,7 | 30,8 | 31,4 |
| F | 41.8 | 2,7 | 4,9 | 3,5 | 3,0 | 3,1 |
| G | 55.74 | 7,6 | 8,4 | 4,9 | 4,6 | 4,5 |
| H | 57.43 | 3,1 | 4,0 | 3,0 | 2,6 | 2,9 |

| | | | | | | |
|---|---|---|---|---|---|---|
| ¹ Berechnet von [³H]-Messwerten. cpm-Messwerte Allergen-stimulierter Zellkulturen/ cpm-Messwerte unstimulierter Zellkulturen ² Donor: Klinisch definierte Gräserpollen-Allergiker | | | | | | |

### Gentechnische Konstruktion von hypoallergenen PhI p 1-Varianten

### Beispiel 1: PhI p 1 NoCys

Zur Konstruktion der Variante PhI p 1 NoCys (SEQ ID NO 3 und 4) wurden sechs PCR-Schritte ausgehend von der cDNA von rPhI p 1 wt ("GenBank" Eintrag Z27090; NCBI, Bethesda, USA) durchgeführt. Die Punktmutationen wurden eingeführt, indem spezifische PCR-Primer eingesetzt wurden, die anstelle von für Cystein kodierenden Kodons solche für Serin enthielten (Primersequenzen s. Tab. 2).

Schritt 1 - Herstellung des N-terminalen DNA-Fragments "PhI p 1 [C41 S, C57S, C69S] (bp 1-212)": Durch Amplifizierung langer überlappender Oligonukleotide (P 1-63, P 49-111, P 97-158 und P 144-212) mittels PCR wurde ein DNA-Fragment mit den Mutationen C41S, C57S, C69S generiert.

Schritt 2 - Herstellung des C-terminalen DNA-Fragments "PhI p 1 [C69S, C72S, C77S, C83S] (bp 193-720)": PCR der PhI p 1 wt-cDNA mit den Primern P 193-261 und P 703-720 *Hind*III.

Schritt 3 - Herstellung der DNA kodierend für "PhI p 1 [C41 S, C57S, C69S, C72S, C77S, C83S] (bp 1-720)": PCR der überlappenden Fragmente "PhI p 1 [C41S, C57S, C69S] (bp 1-212)" und "PhI p 1 [C69S, C72S, C77S, C83S] (bp 193-720)" mit den Primern P 1-63 und P 703-720 *Hind*III.

Schritt 4 - Herstellung des N-terminalen DNA-Fragments "PhI p 1 [C41S, C57S, C69S, C72S, C77S, C83S, C139S] (bp 1-428)": PCR der cDNA von "PhI p 1 [C41S, C57S, C69S, C72S, C77S, C83S] (bp 1-720)" mit den Primern P 1-63 und P 406-428 as.

Schritt 5 - Herstellung des C-terminalen DNA-Fragments "PhI p 1 [C139S] (bp 406-720)": PCR der cDNA von rPhI p 1 wt mit den Primern P 406-428s und P 703-720 *Hind*III.

Schritt 6 - Herstellung der vollständigen DNA kodierend für PhI p 1 NoCys: PCR der überlappenden Fragmente "PhI p 1 [C41 S, C57S, C69S, C72S, C77S, C83S, C139S] (bp 1-428)" und "PhI p 1 [C139S] (bp 406-720) mit den Primern P 1-63 und P 703-720 *Hind*III".

Die für PhI p 1 NoCys kodierende DNA wurden mit dem Restriktionsenzym *Hind*III verdaut und über die Restriktionsschnittstellen *Ehe*I und *Hind*III in den Expressionsvektor pProExHT (Invitrogen, Carlsbad, USA) ligiert, und anschließend vollständig sequenziert.

**Tabelle 2: Aufstellung der zur Herstellung von PhI p 1 NoCys, PhI p 1 NoCys Δ213-220 und PhI p 1 NoCys Δ1-6, 115-119, 213-220 eingesetzten PCR-Primer**

| Primer | Richtung | SEQ ID NO | Sequenz (5'→3') |
|---|---|---|---|
| P 1-18¹ | sense | 19 | atc ccg aag gtc ccg ccg |
| P 1-63 | sense | 20 | |
| P 49-111 | antisense | 21 | |
| P 97-158 | sense | 22 | |
| P 144-212 | antisense | 23 | |
| P 193-261 | sense | 24 | |
| P 703-720 *Hin*dIII | antisense | 25 | ggt aag ctt tca ctt gga ctc gta ggc ggt |
| P 406-428 as | antisense | 26 | tcc ggg tac ttg gac ttg acg cg |
| P 406-428 s | sense | 27 | cgc gtc aag tcc aag tac ccg ga |
| P 22-63 (Δ1-18) | sense | 28 | |
| P 250-318 | antisense | 29 | |
| P 301-384 (Δ343-357) | sense | 30 | |
| P 613-720 (Δ637-660) *Hin*dIII | antisense | 31 | |

| | | | |
|---|---|---|---|
| ¹Zahlenangaben: Positionen der Primer bezogen auf die Nukleotid-Sequenz des PhI p 1 Wildtyp-Proteins (ohne Signalpeptid; "GenBank" Eintrag Z27090; NCBI, Bethesda, USA). Primersequenzen teilweise kodonoptimiert für *E*. *coli.* | | | |

### Beispiel 2: PhI p 1 NoCys Δ213-220

Die DNA-Sequenz kodierend für die Deletionsvariante PhI p 1 NoCys Δ213-220 (SEQ ID NO 15 und 16) wurde mittels PCR der DNA von PhI p 1 NoCys unter Einsatz des 5'-Primers P 1-18 sowie des spezifisch um den zu deletierenden Sequenzbereich verkürzten 3'-Primers P 613-720 (Δ637-660) *Hind*III generiert.
Die cDNA wurden mit dem Restriktionsenzym *Hind*III verdaut und über die Restriktionsschnittstellen *Ehe*I und *Hind*III in den Expressionsvektor pProExHT (Invitrogen, Carlsbad, USA) ligiert, und anschließend vollständig sequenziert.

### Beispiel 3: Gentechnische Konstruktion von PhI p 1 NoCys Δ1-6, 115-119, 213-220

Die DNA-Sequenz kodierend für die Deletionsvariante PhI p 1 NoCys Δ1-6, 115-119, 213-220 (SEQ ID NO 5 und 6) wurde mittels PCR unter Einsatz von spezifisch um den zu deletierenden Sequenzbereich verkürzten Oligonukleotiden in drei Schritten generiert.

Schritt 1 - Herstellung des N-terminalen DNA-Fragments "PhI p 1 NoCys Δ1-6 (bp 1-300)": PCR der cDNA von PhI p 1 NoCys mit den Primern P22-63 (Δ1-18) und P 250-318.

Schritt 2 - Herstellung des C-terminalen DNA-Fragments "PhI p 1 NoCys Δ115-119, 213-220 (bp 283-663)": PCR von PhI p 1 NoCys mit den Primern P 301-384 (Δ1343-357) und P 613-720 (Δ1637-660) *Hind*III.

Schritt 3 - Herstellung der vollständigen DNA kodierend für PhI p 1 NoCys Δ1-6, 115-119, 213-220:
PCR der überlappenden Fragmente "PhI p 1 NoCys Δ1-6 (bp 1-300)" und "PhI p 1 NoCys Δ115-119, 213-220 (bp 283-663)" mit den Primern P22-63 (Δ1-18) und P 703-720 *Hind*III.

Die für PhI p 1 NoCys Δ1-6, 115-119, 213-220 kodierenden DNA wurde mit dem Restriktionsenzym *Hind*III verdaut und über die Restriktionsschnittstellen EheI und *Hind*III in den Expressionsvektor pProExHT (Invitrogen, Carlsbad, USA) ligiert, und anschließend vollständig sequenziert.

Die DNA der Varianten PhI p 1 NoCys Δ1-6 (SEQ ID NO 5 und 6), PhI p 1 NoCys Δ1-30 (SEQ ID NO 7 und 8), PhI p 1 NoCys Δ92-104 (SEQ ID NO 9 und 10), PhI p 1 NoCys Δ115-119 (SEQ ID NO 11 und 12), PhI p 1 NoCys Δ175-185 (SEQ ID NO 13 und 14) wurden in entsprechender Weise hergestellt, kloniert und sequenziert.

## Patentansprüche

1. PhI p 1-Varianten, **dadurch gekennzeichnet dass** sie ein dem Wildtyp-Allergen verringerte IgE-Reaktivität sowie eine weitgehend erhaltene Reaktivität der T-Lymphozyten aufweisen und die Cysteine der dem reifen PhI p 1 Protein entsprechenden Aminosäurepositionen 41, 57, 69, 72, 77, 83 und 139 fehlen.

2. PhI p 1-Varianten gemäß Anspruch 1, **dadurch gekennzeichnet, dass** die Cysteine der dem reifen PhI p 1 Protein entsprechenden Aminosäurepositionen 41, 57, 69, 72, 77, 83 und 139 durch eine andere Aminosäure substituiert werden.

3. PhI p 1-Variante gemäß Anspruch 2, **dadurch gekennzeichnet, dass** die Cysteine der dem reifen PhI p 1 Protein entsprechenden Aminosäurepositionen 41, 57, 69, 72, 77, 83 und 139 durch Serin substituiert werden (Variante PhI p 1 NoCys gemäß SEQ ID NO 4).

4. PhI p 1-Varianten gemäß einem oder mehreren der Ansprüche 1-3, **dadurch gekennzeichnet, dass** mindestens ein Bereich oder eine Kombination von Bereichen, die den Aminosäuren 1-6, 1-30, 92-104, 115-119, 175-185 und 213-220 der Primärsequenz des reifen PhI p 1 Proteins entsprechen, gegenüber dem Wildtyp-Allergen fehlen.

5. PhI p 1-Varianten gemäß einem oder mehreren der Ansprüche 1-4, bei denen die Aminosäuren 213-220 entsprechend der reifen PhI p 1-Sequenz fehlen.

6. PhI p 1-Variante gemäß Anspruch 4 oder 5, bei der die Cysteine 41, 57, 69, 72, 77, 83 und 139 des reifen Proteins durch Serin ersetzt werden und Aminosäuren 213-220 fehlen (Variante PhI p 1 NoCys Δ213-220 gemäß SEQ ID NO 16).

7. PhI p 1-Variante gemäß Anspruch 4, bei denen die Aminosäuren 1-6, 115-119 und 213-220 entsprechend der reifen PhI p 1-Sequenz fehlen.

8. PhI p 1-Variante gemäß Anspruch 4 oder 7, bei der die Cysteine 41, 57, 69, 72, 77, 83 und 139 des reifen Proteins durch Serin ersetzt werden und Aminosäuren 1-6, 115-119 und 213-220 fehlen (Variante PhI p 1 NoCys Δ1-6, 115-119 und 213-220 gemäß SEQ ID NO 18).

9. PhI p 1-Varianten gemäß Anspruch 1-8, **dadurch gekennzeichnet, dass** sie durch gentechnische Methoden rekombinant erhalten wurden.

10. DNA-Moleküle, kodierend für Allergenvarianten gemäß einem oder mehrerer der Ansprüche 1-9.

11. Rekombinanter Expressionsvektor, enthaltend ein DNA-Molekül gemäß Anspruch 10, funktionell verbunden mit einer Expressions-Kontrollsequenz.

12. Nicht-menschlicher Wirtsorganismus, transformiert mit einem DNA-Molekül gemäß Anspruch 10 oder einem Expressionsvektor gemäß Anspruch 11.

13. Verfahren zur Herstellung einer Allergenvariante gemäß einem oder mehrerer der Ansprüche 1-9 durch Kultivierung eines Wirtsorganismus gemäß Anspruch 12 und Gewinnung der entsprechenden Allergenvariante aus der Kultur.

14. Allergenvariante gemäß einem oder mehrerer der Ansprüche 1-9 als Arzneimittel.

15. Pharmazeutische Zubereitung, enthaltend mindestens eine Allergenvariante entsprechend einem oder mehreren der Ansprüche 1-9 und gegebenenfalls weitere Wirk- und/oder Hilfsstoffe zur präventiven und therapeutischen Behandlung von Allergien an deren Auslösung Gruppe 1-Allergene der *Poaceae* Spezies beteiligt sind.

16. Verwendung mindestens einer Allergenvariante gemäß einem oder mehreren der Ansprüche 1-9 zur Herstellung eines Arzneimittels zur Prävention und Therapie von Allergien an deren Auslösung Gruppe 1-Allergene von Spezies der *Poaceae* beteiligt sind.

17. DNA-Molekül gemäß Anspruch 10 als Arzneimittel.

18. Rekombinanter Expressionsvektor gemäß Anspruch 11 als Arzneimittel.

19. Pharmazeutische Zubereitung, enthaltend mindestens ein DNA-Molekül gemäß Anspruch 10 oder mindestens ein Expressionsvektor gemäß Anspruch 18 und gegebenenfalls weitere Wirk- und/oder Hilfsstoffe zur immuntherapeutischen DNA-Vakzinierung von Patienten mit Allergien, an deren Auslösung Gruppe 1-Allergene der *Poaceae* beteiligt sind und/oder zur Prävention solcher Allergien.

20. Verwendung mindestens eines DNA-Moleküls gemäß Anspruch 10 oder mindestens eines Expressionsvektors gemäß Anspruch 18 zur Herstellung eines Arzneimittels zur immuntherapeutischen DNA-Vakzinierung von Patienten mit Allergien, an deren Auslösung Gruppe 1-Allergene der *Poaceae* beteiligt sind und/oder zur Prävention solcher Allergien.

## Claims

1. PhI p 1 variants, **characterised in that** they have reduced IgE reactivity compared with the wild-type allergen and substantially maintained reactivity of the T-lymphocytes, and the cysteines at amino acid positions 41, 57, 69, 72, 77, 83 and 139 corresponding to the mature PhI p 1 protein are missing.

2. PhI p 1 variants according to Claim 1, **characterised in that** the cysteines at amino acid positions 41, 57, 69, 72, 77, 83 and 139 corresponding to the mature PhI p 1 protein are substituted by another amino acid.

3. PhI p 1 variant according to Claim 2, **characterised in that** the cysteines at amino acid positions 41, 57, 69, 72, 77, 83 and 139 corresponding to the mature PhI p 1 protein are substituted by serine (variant PhI p 1 NoCys according to SEQ ID NO 4).

4. PhI p 1 variants according to one or more of Claims 1-3, **characterised in that** at least one region or a combination of regions which correspond to amino acids 1-6, 1-30, 92-104, 115-119, 175-185 and 213-220 of the primary sequence of the mature PhI p 1 protein are missing compared with the wild-type allergen.

5. PhI p 1 variants according to one or more of Claims 1-4 in which amino acids 213-220 corresponding to the mature PhI p 1 sequence are missing.

6. PhI p 1 variant according to Claim 4 or 5 in which cysteines 41, 57, 69, 72, 77, 83 and 139 of the mature protein are replaced by serine and amino acids 213-220 are missing (variant PhI p 1 NoCys Δ213-220 according to SEQ ID NO 16).

7. PhI p 1 variant according to Claim 4 in which amino acids 1-6, 115-119 and 213-220 corresponding to the mature PhI p 1 sequence are missing.

8. PhI p 1 variant according to Claim 4 or 7 in which cysteines 41, 57, 69, 72, 77, 83 and 139 of the mature protein are replaced by serine and amino acids 1-6, 115-119 and 213-220 are missing (variant PhI p 1 NoCys Δ1-6, 115-119 and 213-220 according to SEQ ID NO 18).

9. PhI p 1 variants according to Claims 1-8, **characterised in that** they have been obtained by recombinant genetic engineering methods.

10. DNA molecules encoding for allergen variants according to one or more of Claims 1-9.

11. Recombinant expression vector comprising a DNA molecule according to Claim 10 functionally connected to an expression control sequence.

12. Non-human host organism transformed by means of a DNA molecule according to Claim 10 or an expression vector according to Claim 11.

13. Process for the preparation of an allergen variant according to one or more of Claims 1-9 by cultivation of a host organism according to Claim 12 and isolation of the corresponding allergen variant from the culture.

14. Allergen variant according to one or more of Claims 1-9 as medicament.

15. Pharmaceutical composition comprising at least one allergen variant corresponding to one or more of Claims 1-9 and optionally further active compounds and/or auxiliaries for the preventive and therapeutic treatment of allergies in the triggering of which group 1 allergens of *Poaceae* species are involved.

16. Use of at least one allergen variant according to one or more of Claims 1-9 for the preparation of a medicament for the prevention and therapy of allergies in the triggering of which group 1 allergens from *Poaceae* species are involved.

17. DNA molecule according to Claim 10 as medicament.

18. Recombinant expression vector according to Claim 11 as medicament.

19. Pharmaceutical composition comprising at least one DNA molecule according to Claim 10 or at least one expression vector according to Claim 18 and optionally further active compounds and/or auxiliaries for the immunotherapeutic DNA vaccination of patients having allergies in the triggering of which group 1 allergens of the *Poaceae* are involved and/or for the prevention of such allergies.

20. Use of at least one DNA molecule according to Claim 10 or at least one expression vector according to Claim 18 for the preparation of a medicament for the immunotherapeutic DNA vaccination of patients having allergies in the triggering of which group 1 allergens of the *Poaceae* are involved and/or for the prevention of such allergies.

## Revendications

1. Variants de PhI p 1**, caractérisés en ce qu'**ils ont une réactivité d'IgE réduite par rapport à l'allergène de type sauvage et une réactivité des lymphocytes T essentiellement maintenue, et les cystéines au niveau des positions d'acides aminés 41, 57, 69, 72, 77, 83 et 139 correspondant à la protéine PhI p 1 mature sont absentes.

2. Variants de PhI p 1 selon la revendication 1, **caractérisés en ce que** les cystéines au niveau des positions d'acides aminés 41, 57, 69, 72, 77, 83 et 139 correspondant à la protéine PhI p 1 mature sont substituées par un autre acide aminé.

3. Variant de PhI p 1 selon la revendication 2, **caractérisé en ce que** les cystéines au niveau des positions d'acides aminés 41, 57, 69, 72, 77, 83 et 139 correspondant à la protéine PhI p 1 mature sont substituées par la sérine (variant de PhI p 1 NoCys selon SEQ ID n° 4).

4. Variants de PhI p 1 selon l'une ou plusieurs des revendications 1-3, **caractérisés en ce qu'**au moins une région ou une combinaison de régions correspondant aux acides aminés 1-6, 1-30, 92-104, 115-119, 175-185 et 213-220 de la séquence primaire de la protéine PhI p 1 mature sont absentes par rapport à l'allergène de type sauvage.

5. Variants de PhI p 1 selon l'une ou plusieurs des revendications 1-4, dans lesquels les acides aminés 213-220 correspondant à la séquence de PhI p 1 mature sont absents.

6. Variant de PhI p 1 selon la revendication 4 ou 5, dans lequel les cystéines 41, 57, 69, 72, 77, 83 et 139 de la protéine mature sont remplacées par la sérine et les acides aminés 213-220 sont absents (variant de PhI p 1 NoCys Δ213-220 selon SEQ ID n° 16).

7. Variant de PhI p 1 selon la revendication 4, dans lequel les acides aminés 1-6, 115-119 et 213-220 correspondant à la séquence de Phl p 1 mature sont absents.

8. Variant de PhI p 1 selon la revendication 4 ou 7, dans lequel les cystéines 41, 57, 69, 72, 77, 83 et 139 de la protéine mature sont remplacées par la sérine et les acides aminés 1-6, 115-119 et 213-220 sont absents (variant de PhI p 1 NoCys Δ1-6, 115-119 et 213-220 selon SEQ ID n° 18).

9. Variants de PhI p 1 selon les revendications 1-8, **caractérisés en ce qu'**ils ont été obtenus par des méthodes de génie génétique par recombinaison.

10. Molécules d'ADN codant pour des variants allergènes selon l'une ou plusieurs des revendications 1-9.

11. Vecteur d'expression recombinant comprenant une molécule d'ADN selon la revendication 10 liée de manière fonctionnelle à une séquence de contrôle d'expression.

12. Organisme hôte non humain transformé au moyen d'une molécule d'ADN selon la revendication 10 ou d'un vecteur d'expression selon la revendication 11.

13. Procédé de préparation d'un variant allergène selon l'une ou plusieurs des revendications 1-9 par la mise en culture d'un organisme hôte selon la revendication 12 et l'isolement du variant allergène correspondant à partir de la culture.

14. Variant allergène selon l'une ou plusieurs des revendications 1-9 comme médicament.

15. Composition pharmaceutique comprenant au moins un variant allergène correspondant à l'une ou plusieurs des revendications 1-9 et éventuellement d'autres composés actifs et/ou auxiliaires pour le traitement préventif et thérapeutique d'allergies dont le déclenchement met en jeu les allergènes de groupe 1 des espèces *Poaceae*.

16. Utilisation d'au moins un variant allergène selon l'une ou plusieurs des revendications 1-9 pour la préparation d'un médicament destiné à la prévention et la thérapie d'allergies dont le déclenchement met en jeu les allergènes de groupe 1 des espèces *Poaceae.*

17. Molécule d'ADN selon la revendication 10 comme médicament.

18. Vecteur d'expression recombinant selon la revendication 11 comme médicament.

19. Composition pharmaceutique comprenant au moins une molécule d'ADN selon la revendication 10 ou au moins un vecteur d'expression selon la revendication 18 et éventuellement d'autres composés actifs et/ou auxiliaires pour la vaccination immunothérapeutique par ADN de patients atteints d'allergies dont le déclenchement met en jeu les allergènes de groupe 1 des *Poaceae* et/ou pour la prévention de telles allergies.

20. Utilisation d'au moins une molécule d'ADN selon la revendication 10 ou d'au moins un vecteur d'expression selon la revendication 18 pour la préparation d'un médicament destiné à la vaccination immunothérapeutique par ADN de patients atteints d'allergies dont le déclenchement met en jeu les allergènes de groupe 1 des *Poaceae* et/ou pour la prévention de telles allergies.
